# EUROPEAN PATENT APPLICATION

(11) **EP 4 595 968 A1**
(43) Date of publication of application: **06.08.2025**
(21) Application number: 23869844.3
(22) Date of filing: 30.06.2023
(51) Int. Cl.: A61K 36/8968, A61P 7/04, A61P 15/00, G01N 30/06, G01N 30/86, G01N 33/15, A61K 35/36

(54) **REHMANNIA AND LYCIUM ROOT BARK DECOCTION WATER EXTRACT SOLUTION, REHMANNIA AND LYCIUM ROOT BARK DECOCTION WATER EXTRACT PASTE, REHMANNIA AND LYCIUM ROOT BARK DECOCTION PREPARATION AND PREPARATION METHOD THEREFOR, AND QUALITY CONTROL STANDARD FOR REHMANNIA AND LYCIUM ROOT BARK DECOCTION PREPARATION**

(30) Priority: 26.09.2022 CN 202211173779
(71) Applicant: Dong-e E-jiao Co., Ltd., Liaocheng, Shandong 252200 (CN)
(72) Inventor: WANG, Chunyan, Liaocheng, Shandong 252200 (CN); LIU, Haibin, Liaocheng, Shandong 252200 (CN); ZHANG, Yan, Liaocheng, Shandong 252200 (CN); WANG, Zhongchao, Liaocheng, Shandong 252200 (CN); CHENG, Jie, Liaocheng, Shandong 252200 (CN); QI, Xiaodan, Liaocheng, Shandong 252200 (CN); KONG, Lingmei, Liaocheng, Shandong 252200 (CN); QIAN, Liyan, Liaocheng, Shandong 252200 (CN); MA, Liping, Liaocheng, Shandong 252200 (CN); ZHANG, Yan, Liaocheng, Shandong 252200 (CN); ZHAO, Haiqing, Liaocheng, Shandong 252200 (CN); YANG, Haiju, Liaocheng, Shandong 252200 (CN)
(74) Representative: Plougmann Vingtoft a/s
(86) International application number: PCT/CN2023/104552
(87) International publication number: WO 2024/066596

(57) **Abstract**

Provided are a rehmannia and lycium root bark decoction water extract solution, a rehmannia and lycium root bark decoction water extract paste, a rehmannia and lycium root bark decoction preparation and a preparation method therefor, and a detection method for a rehmannia and lycium root bark decoction preparation. Active pharmaceutical ingredients of the rehmannia and lycium root bark decoction are rehmannia stir-baked with wine, radix scrophulariae, radix paeoniae alba stir-baked with wine, radix ophiopogonis, lycium root bark, and donkey-hide gelatin. According to the preparation method, a concentration approach in which extract solutions obtained from three extractions are concentrated separately is used, such that the concentration time of paeoniflorin at a low concentration and the absolute heating concentration time of a herb solution are shortened, thereby solving the problem of degradation of the active ingredient paeoniflorin in the rehmannia and lycium root bark decoction. The concentration after extraction is carried out at a relatively low temperature and in vacuum, such that protection and retention of the ingredients are facilitated, avoiding the degradation of ingredients such as paeoniflorin caused by a high temperature.

## Description

The present application claims priority to the Chinese Patent Application CN202211173779.8 filed with the China National Intellectual Property Administration (CNIPA) on September 26, 2022 and entitled "Shuandi decoction water extract, Shuandi decoction water extract paste, Shuandi decoction preparation and preparation method thereof, and quality control standard of Shuandi decoction preparation", which is incorporated herein by reference in its entirety.

### TECHNICAL FIELD

The present disclosure relates to the technical field of medicine, and specifically relates to a Liang Di Tang water-extracted liquid, a Liang Di Tang water-extracted paste, and a Liang Di Tang preparation, and preparation methods thereof, and a quality control standard for the Liang Di Tang preparation.

### BACKGROUND

Liang Di Tang is the 90th formula listed in the *Catalogue of Ancient Classical Formulas* (first batch) issued by the China National Administration of Traditional Chinese medicine. Liang Di Tang originates from the *Fu Qing-Zhu's Gynecology* (Fu Shan, Qing Dynasty). Liang Di Tang is composed of the following six traditional Chinese medicines: wine-processed *Rehmanniae Radix, Scrophulariae Radix,* wine-processed *Paeoniae Radix Alba, Ophiopogon japonicus, Lycii Cortex,* and *Asini Corii Colla.* Liang Di Tang is a classical formula for menstrual regulation in gynaecology, which mainly has the therapeutic effect of nourishing yin and clearing heat, and cooling blood and normalizing menstrual cycle. The major indications of Liang Di Tang are early menstruation, scanty menstrual flow, prolonged menstrual cycle, dysfunctional uterine bleeding, and other symptoms caused by yin deficiency with intrinsic heat.

CN110412155A discloses a method for testing a high-performance liquid chromatography (HPLC) characteristic chromatogram of Liang Di Tang. Paeoniflorin and harpagoside are adopted as reference substances. CN110412155A also discloses a method for preparing Liang Di Tang, including the following steps: 37.3 g of wine-processed *Rehmanniae Radix,* 37.3 g of *Scrophulariae Radix,* 18.6 g of wine-processed *Paeoniae Radix Alba,* 18.6 g of *Ophiopogon japonicus,* and 11.2 g of *Lycii Cortex* are added to water, soaked for 40 min, and a first extraction and a second extraction are conducted while covered to produce extraction solutions. The first extraction is conducted for 30 min with water at an amount 7 times a total amount of the above five traditional Chinese medicines, and the second extraction is conducted for 20 min with water at an amount 5 times the total amount of the five traditional Chinese medicines. The extraction solutions are decocted and filtered to produce filtrates, and the filtrates are combined and concentrated to about 500 mL. 11.2 g of *Asini Corii Colla* is added thereto and Yanghua (being melted by gentle heating) to produce a standard decoction. The standard decoction is vacuum-concentrated at a low temperature to about 250 mL, and then lyophilized to produce a lyophilized powder. However, in the above preparation method of Liang Di Tang, the filtrates are combined and then concentrated, and the concentration time is long. As a result, the heat-sensitive active ingredients such as paeoniflorin in Liang Di Tang are easily decomposed, which adversely affects the therapeutic efficacy of Liang Di Tang.

### SUMMARY

In view of this, an object of the present disclosure is to provide a Liang Di Tang water-extracted liquid, a Liang Di Tang water-extracted paste, and a Liang Di Tang preparation, and preparation methods thereof, and a quality control standard for the Liang Di Tang preparation. The Liang Di Tang water-extracted liquid, the Liang Di Tang water-extracted paste, and the Liang Di Tang preparation provided by the present disclosure have high contents of heat-sensitive active ingredients such as paeoniflorin, and exhibit excellent efficacy.

To achieve the above object of the present disclosure, the present disclosure provides the following technical solutions:
The present disclosure provides a method for preparing a Liang Di Tang water-extracted liquid, including the following steps:
mixing five traditional Chinese medicines with a first water, and conducting a first soaking and then a first extraction to obtain a first extraction solution and a first medicinal residue, where the five traditional Chinese medicines are wine-processed *Rehmanniae Radix, Scrophulariae Radix,* wine-processed *Paeoniae Radix Alba, Ophiopogon japonicus,* and *Lycii Cortex*;
mixing the first medicinal residue with a second water, and conducting a second soaking and then a second extraction to obtain a second extraction solution and a second medicinal residue;
mixing the second medicinal residue with a third water, and conducting a third soaking and then a third extraction to obtain a third extraction solution;
concentrating the first extraction solution, the second extraction solution, and the third extraction solution separately to obtain a first concentrate, a second concentrate, and a third concentrate, respectively, where the concentrating is conducted at a temperature independently of 70 °C to 90 °C, a vacuum degree independently of 0.03 MPa to 0.07 MPa, and a steam pressure independently of not larger than 0.09 MPa; and
mixing the first concentrate, the second concentrate, the third concentrate, and an *Asini Corii Colla* liquid to obtain the Liang Di Tang water-extracted liquid,
where a mass ratio of the wine-processed *Rehmanniae Radix,* the *Scrophulariae Radix,* the wine-processed *Paeoniae Radix Alba,* the *Ophiopogon japonicus,* the *Lycii Cortex,* and *Asini Corii Colla* is 37.30 : 37.30 : 18.65 : 18.65 : 11.19 : 11.19.

In some embodiments, a mass of the first water is 6 times to 8 times a total mass of the five traditional Chinese medicines;
a mass of the second water and a mass of the third water each are independently 4 times to 6 times the total mass of the five traditional Chinese medicines;
the first extraction, the second extraction, and the third extraction each are conducted independently at a temperature of 98 °C to 101 °C;
the first soaking and the first extraction each are conducted independently for 30 min to 50 min; and
the second soaking, the second extraction, the third soaking, and the third extraction each are conducted independently for 20 min to 40 min.

In some embodiments, the first concentrate, the second concentrate, and the third concentrate each have a density independently of greater than or equal to 1.10 g/mL and less than 1.2 g/mL.

In some embodiments, the *Asini Corii Colla* liquid is prepared by a process including the following steps: placing the *Asini Corii Colla* in water, liquefying the *Asini Corii Colla,* and sieving to obtain an undersized part, namely the *Asini Corii Colla* liquid, where a mass of the water is 2 times to 6 times a mass of the *Asini Corii Colla,* and a sieve used for the sieving has a sieve size of 100 mesh.

The present disclosure provides a Liang Di Tang water-extracted liquid prepared by the method as described in the above technical solutions.

The present disclosure provides a Liang Di Tang water-extracted paste obtained by concentrating the Liang Di Tang water-extracted liquid as described in the above technical solutions, where the Liang Di Tang water-extracted paste has a density of 1.2 g/mL to 1.3 g/mL.

The present disclosure provides a Liang Di Tang preparation prepared from raw materials including a Liang Di Tang water-extracted extract and a pharmaceutically acceptable adjuvant, where the Liang Di Tang water-extracted extract is at least one selected from the group consisting of the Liang Di Tang water-extracted liquid as described in the above technical solutions and the Liang Di Tang water-extracted paste as described in the above technical solutions; and a mass ratio of the pharmaceutically acceptable adjuvant to the Liang Di Tang water-extracted extract is in a range of 1 : (12.48-18.72), and a mass of the Liang Di Tang water-extracted extract is based on a total mass of wine-processed *Rehmanniae Radix, Scrophulariae Radix,* wine-processed *Paeoniae Radix Alba, Ophiopogon japonicus, Lycii Cortex,* and *Asini Corii Colla.*

In some embodiments, in parts by mass, the raw materials for preparing 1,000 parts of the Liang Di Tang preparation include: in parts by mass, raw materials for preparing 1,000 parts of the Liang Di Tang preparation include: 468 parts to 572 parts of the wine-processed *Rehmanniae Radix,* 468 parts to 572 parts of the *Scrophulariae Radix,* 234 parts to 286 parts of the wine-processed *Paeoniae Radix Alba,* 234 parts to 286 parts of the *Ophiopogon japonicus,* 140 parts to 171 parts of the *Lycii Cortex,* 140 parts to 171 parts of the *Asini Corii Colla,* and 100 parts to 150 parts of the pharmaceutically acceptable adjuvant.

The present disclosure provides a method for preparing the Liang Di Tang preparation as described in the above technical solutions, including the following steps:
mixing and concentrating the Liang Di Tang water-extracted extract and an aqueous solution of the pharmaceutically acceptable adjuvant to obtain a mixed concentrate; and
subjecting the mixed concentrate to vacuum belt drying to obtain the Liang Di Tang preparation.

The present disclosure provides a quality control standard for the Liang Di Tang preparation as described in the above technical solutions or the Liang Di Tang preparation prepared by the method as described in the above technical solutions, including an HPLC characteristic chromatogram, characteristic component contents, a water content, and a dry extract yield of the Liang Di Tang preparation;
the characteristic component contents include: a harpagoside content of 0.33 mg/g to 0.67 mg/g, a paeoniflorin content of 2.62 mg/g to 4.96 mg/g, an L-hydroxyproline content of 8.83 mg/g to 16.42 mg/g, and a glycine content of 17.42 mg/g to 32.46 mg/g;
the water content ranges from 3 wt% to 8 wt%; and
the dry extract yield ranges from 40% to 55%.

In the method for preparing the Liang Di Tang water-extracted liquid provided by the present disclosure, the concentration after extraction is conducted at a low temperature (70 °C to 90 °C) under vacuum (a vacuum degree of 0.03 MPa to 0.07 MPa, and a steam pressure of not larger than 0.09 MPa), which is conducive to the protection and retention of components and could avoid the destruction of ingredients (heat-sensitive active ingredients such as paeoniflorin) due to high temperature. In the traditional concentration for Chinese patent drugs, the extraction solutions produced after several extractions are combined and then concentrated to a specified density, which reduces the total concentration of a medicinal liquid and prolongs the absolute heating and concentration time for the medicinal liquid. In the present disclosure, the three extraction solutions produced after three extractions are concentrated separately, such that a content of paeoniflorin in each of the three extraction solutions quickly reaches a high level, which significantly reduces the concentration time of paeoniflorin at a low content (an environment where paeoniflorin is easily destructed) and the absolute heating and concentration time of a medicinal liquid, solves the problem that the pharmacodynamic ingredient paeoniflorin in Liang Di Tang is easily destructed, and ensures the consistency between the traditional decoction and the modern preparation. As a result, the Liang Di Tang water-extracted liquid produced in the present disclosure has high contents of effective active ingredients and excellent efficacy.

In the modern production of Chinese patent drugs, the extraction rate is blindly pursued, and the extraction each time is often conducted for 1 h to 2 h. However, additional extracted components are often some impurities such as fibers and starches, and the extraction of effective ingredients would not increase. Moreover, the long-term heating may even cause the destruction of effective ingredients. Compared with the modern extraction modes for Chinese patent drugs that involve excessive extraction and high energy consumption, the preparation method provided by the present disclosure allows a moderate water amount, a short extraction time, and a high extraction efficiency (a high extract yield, and high contents of paeoniflorin and harpagoside) during the extraction, which further improves the quality and efficacy of a Liang Di Tang product and reduces the production cost.

Because Liang Di Tang includes heat-sensitive ingredients such as *Asini Corii Colla* (highly viscous and easy to agglomerate), polysaccharides, and paeoniflorin, the traditional high-temperature drying and spray-drying modes are easy to cause agglomeration and may cause destruction to heat-sensitive active ingredients such as paeoniflorin. The method for preparing the Liang Di Tang preparation provided by the present disclosure adopts a vacuum belt drying mode, which has advantages such as low drying temperature, short drying time, small loss, and high efficiency. During the drying process, the Liang Di Tang preparation is not prone to agglomeration, and the heat-sensitive ingredients such as paeoniflorin are not destroyed. As a result, the components are evenly distributed in the Liang Di Tang preparation, and the Liang Di Tang preparation has high contents of active ingredients such as paeoniflorin and exhibits prominent efficacy.

The present disclosure also provides a quality control standard for the Liang Di Tang preparation as described in the above technical solutions or the Liang Di Tang preparation prepared by the method as described in the above technical solutions. The present disclosure adopts a plurality of component indexes (a paeoniflorin content, a harpagoside content, a water content, and a dry extract yield) and a characteristic chromatogram to comprehensively monitor the overall chemical composition of the Liang Di Tang preparation, which involves the comprehensive quality standard and is of great significance for ensuring the stability and high quality of a Liang Di Tang preparation product.

### BRIEF DESCRIPTION OF THE DRAWINGS

FIG. 1 shows an HPLC reference characteristic chromatogram of a Liang Di Tang reference sample according to an embodiment of the present disclosure;
FIG. 2 shows an HPLC reference characteristic chromatogram of a Liang Di Tang preparation according to an embodiment of the present disclosure;
FIG. 3 shows water absorption curves of Chinese herbal slices for Liang Di Tang;
FIG. 4 shows dissolution curves of harpagoside;
FIG. 5 shows dissolution curves of paeoniflorin;
FIG. 6 shows liquid chromatography chromatograms of paeoniflorin in test sample solutions of series 1;
FIG. 7 shows liquid chromatography chromatograms of paeoniflorin in test sample solutions of series 2;
FIG. 8 shows liquid chromatography chromatograms of paeoniflorin in test sample solutions of series 3;
FIG. 9 shows liquid chromatography chromatograms of paeoniflorin in test sample solutions of series 4;
FIG. 10 shows liquid chromatography chromatograms of paeoniflorin in test sample solutions of series 5;
FIG. 11 shows test results of changes of paeoniflorin solutions at different concentrations with a heating time;
FIG. 12 shows chromatographic changes of a paeoniflorin solution at a concentration of 0.05 mg/mL when heated for 4 h; and
FIG. 13 shows chromatographic changes of paeoniflorin solutions at different concentrations when heated for 4 h.

### DETAILED DESCRIPTION OF THE EMBODIMENTS

The present disclosure provides a method for preparing a Liang Di Tang water-extracted liquid, including the following steps:
mixing five traditional Chinese medicines with a first water, and conducting a first soaking and then a first extraction to obtain a first extraction solution and a first medicinal residue, wherein the five traditional Chinese medicines are wine-processed *Rehmanniae Radix, Scrophulariae Radix,* wine-processed *Paeoniae Radix Alba, Ophiopogon japonicus,* and *Lycii Cortex*;
mixing the first medicinal residue with a second water, and conducting a second soaking and then a second extraction to obtain a second extraction solution and a second medicinal residue;
mixing the second medicinal residue with a third water, and conducting a third soaking and then a third extraction to obtain a third extraction solution;
concentrating the first extraction solution, the second extraction solution, and the third extraction solution separately to obtain a first concentrate, a second concentrate, and a third concentrate, respectively, wherein the concentrating is conducted at a temperature independently of 70 °C to 90 °C, a vacuum degree independently of 0.03 MPa to 0.06 MPa, and a steam pressure independently of 0.03 MPa to 0.05 MPa; and
mixing the first concentrate, the second concentrate, the third concentrate, and an *Asini Corii Colla* liquid to obtain the Liang Di Tang water-extracted liquid,
where a mass ratio of the wine-processed *Rehmanniae Radix,* the *Scrophulariae Radix,* the wine-processed *Paeoniae Radix Alba,* the *Ophiopogon japonicus,* the *Lycii Cortex,* and *Asini Corii Colla* is 37.30 : 37.30 :18.65 : 18.65 : 11.19 : 11.19.

In the present disclosure, unless otherwise specified, all raw material components are commercially-available products well known to those skilled in the art.

In the present disclosure, five traditional Chinese medicines are mixed with the first water, and the first soaking and the first extraction are conducted sequentially to obtain the first extraction solution and the first medicinal residue. The five traditional Chinese medicines are wine-processed *Rehmanniae Radix, Scrophulariae Radix,* wine-processed *Paeoniae Radix Alba, Ophiopogon japonicus,* and *Lycii Cortex.* In the present disclosure, a mass ratio of the wine-processed *Rehmanniae Radix,* the *Scrophulariae Radix,* the wine-processed *Paeoniae Radix Alba,* the *Ophiopogon japonicus,* and the *Lycii Cortex* is 37.3 : 37.3 : 18.65 : 18.65 : 11.9.

In some embodiments of the present disclosure, the five traditional Chinese medicines are processed (Paozhi, processing of traditional Chinese medicines) before use. In some embodiments of the present disclosure, processing the wine-processed *Rehmanniae Radix* includes the following steps: (1) selection: removing impurities; (2) spray-washing: spray-washing for 10 min to 15 min; (3) enclosed moistening: enclosed moistening at a temperature of 15 °C to 35 °C for 8 h to 12 h; (4) cutting: with a gap between a blade and an edge being set to 3 mm, cutting into thick slices of 2 mm to 4 mm; (5) drying: with a layer thickness of 2 cm to 3 cm and a temperature of 60 °C to 70 °C, drying for 6 h to 7 h; (6) wine-frying: adding 15% yellow rice wine, and frying at 220 °C for 24 min until a surface temperature of the medicine is a range of 130 °C to 135 °C; and (7) refined selection: sieving with a 3-mm medicinal sieve to remove debris, and manually discarding unqualified slices that are charred or have an unqualified thickness. In some embodiments of the present disclosure, processing the *Scrophulariae Radix* includes the following steps: (1) selection: removing impurities; (2) spray-washing: spray-washing for 15 min to 20 min; (3) enclosed moistening: enclosed moistening at a temperature of 5 °C to 35 °C for 4 h to 26 h; (4) cutting: with a gap between a blade and an edge being set to 1.5 mm, cutting into thin slices of 1 mm to 2 mm; (5) drying: with a layer thickness of 2 cm to 3 cm and a temperature of 50 °C to 60 °C, drying for 3.5 h to 4.5 h; and (6) refined selection: sieving with a 2-mm medicinal sieve to remove debris, and manually discarding unqualified slices that are charred or have an unqualified thickness. In some embodiments of the present disclosure, processing the wine-processed *Paeoniae Radix Alba* includes the following steps: (1) selection: removing impurities and grading (small grade: a diameter of a root tip being smaller than 1.0 cm; and large grade: a diameter of a root tip being larger than or equal to 1.0 cm); (2) spray-washing: spray-washing for 10 min to 15 min; (3) soaking: at a temperature of 15 °C to 35 °C, soaking medicine(s) at the small grade for 12 h to 40 h, and medicine(s) at the large grade for 24 h to 56 h; (4) cutting: with a gap between a blade and an edge being set to 1.5 mm, cutting into thick slices of 1 mm to 2 mm; (5) drying: with a layer thickness of 2 cm to 3 cm and a temperature of 70 °C to 80 °C, drying for 4 h to 5 h; (6) wine-frying: adding 20% yellow rice wine, and frying at 230 °C for 20 min until a surface temperature of the medicine(s) is 140 °C to 145 °C; and (7) refined selection: sieving with a 3-mm medicinal sieve to remove debris, and manually discarding unqualified slices that are charred or have an unqualified thickness. In some embodiments of the present disclosure, processing the *Ophiopogon japonicus* includes the following steps: (1) selection: removing impurities; (2) spray-washing: spray-washing for 10 min to 15 min; (3) enclosed moistening: enclosed moistening at a temperature of 5 °C to 35 °C for 6 h to 24 h; (4) flattening: with a roller clearance of 2 mm to 3 mm, pressing medicine(s) into flattened pieces; (5) drying: with a layer thickness of 2 cm to 3 cm and a temperature of 50 °C to 60 °C, drying for 6 h to 7 h; and (6) refined selection: manually discarding unqualified products that are not flattened. In some embodiments of the present disclosure, processing the *Lycii Cortex* includes the following steps: (1) selection: removing impurities and residual woody cores. (2) water-washing: washing with water until there is a natural color and there are free of dust impurities; (3) drying: with a layer thickness of 2 cm to 3 cm and a temperature of 40 °C to 50 °C, drying for 4 h to 5 h; and (4) sieving: sieving with a 3-mm medicinal sieve to remove debris.

In some embodiments of the present disclosure, a mass of the first water is 6 times to 8 times, preferably 6 times to 7.5 times, and more preferably 6.5 times to 7 times a mass of the five traditional Chinese medicines. In some embodiments of the present disclosure, the first soaking is conducted at room temperature; the first soaking is conducted for preferably 30 min to 50 min and more preferably 35 min to 40 min. In some embodiments of the present disclosure, the first extraction is conducted at a temperature of 98 °C to 101 °C and preferably 99 °C to 100 °C; the first extraction is conducted for preferably 30 min to 50 min and more preferably 35 min to 40 min.

In some embodiments of the present disclosure, after the first extraction is completed, the method further includes subjecting a first extraction system obtained to post-treatment. In some embodiments, the post-treatment includes: subjecting the first extraction system to centrifugal separation to obtain a liquid component and the first medicinal residue, and subjecting the liquid component to fine filtration to obtain the first extraction solution. In some embodiments of the present disclosure, the centrifugal separation is carried out in a centrifuge. In the present disclosure, there is no special limitation on conditions of the centrifugal separation, as long as the liquid component and the solid component could be separated from each other. In some embodiments of the present disclosure, the fine filtration is conducted with a plate and frame filter, and a filter membrane of the plate and frame filter has a pore size preferably of 5 µm to 15 µm.

In the present disclosure, after the first medicinal residue is obtained, the first medicinal residue is mixed with the second water, and the second soaking and the second extraction are conducted sequentially to obtain the second extraction solution and the second medicinal residue. In some embodiments of the present disclosure, a mass of the second water is 4 times to 6 times, preferably 4 times to 5.5 times, and more preferably 4.5 times to 5 times the mass of the five traditional Chinese medicines. In some embodiments of the present disclosure, the second soaking is conducted at room temperature; the second extraction is conducted preferably at the same temperature as the first extraction, which will not be repeated here; the second soaking and the second extraction each are conducted independently for preferably 20 min to 40 min, more preferably 20 min to 35 min, and further more preferably 25 min to 30 min. In some embodiments of the present disclosure, after the second extraction is completed, the method further includes subjecting a second extraction system obtained to post-treatment; the post-treatment for the second extraction system is the same as the post-treatment for the first extraction system, which will not be repeated here.

In the present disclosure, after the second medicinal residue is obtained, the second medicinal residue is mixed with the third water, and the third soaking and the third extraction are conducted sequentially to obtain the third extraction solution. In some embodiments of the present disclosure, a mass of the third water is 4 times to 5 times and more preferably 4.5 times the mass of the five traditional Chinese medicines. In some embodiments of the present disclosure, the third soaking is conducted at room temperature; the third extraction is conducted preferably at the same temperature as the first extraction, which will not be repeated here; the third soaking and the third extraction each are conducted independently for preferably 20 min to 40 min, preferably 20 min to 35 min, and further more preferably 25 min to 30 min. In some embodiments of the present disclosure, after the third extraction is completed, the method further includes subjecting a second extraction system obtained to post-treatment; the post-treatment for the second extraction system is the same as the post-treatment for the first extraction system, which will not be repeated here.

In the present disclosure, after the first extraction solution, the second extraction solution, and the third extraction solution are obtained, the first extraction solution, the second extraction solution, and the third extraction solution are concentrated separately to produce the first concentrate, the second concentrate, and the third concentrate, respectively. In some embodiments of the present disclosure, a first concentration, a second concentration, and a third concentration each are conducted at a temperature of 70 °C to 90 °C, preferably 75 °C to 85 °C, and more preferably 80 °C, a vacuum degree independently of 0.03 MPa to 0.07 MPa and preferably 0.04 MPa to 0.06 MPa, and a steam pressure independently of lower than or equal to 0.09 MPa and preferably 0.03 MPa to 0.05 MPa. In the present disclosure, there is no special limitation on a time for each of the first concentration, the second concentration, and the third concentration, as long as the first concentrate, the second concentrate, and the third concentrate produced after concentration each have a density independently of greater than or equal to 1.10 g/mL and less than 1.2 g/mL.

In the present disclosure, after the first concentrate, the second concentrate, and the third concentrate are obtained, the first concentrate, the second concentrate, the third concentrate, and an *Asini Corii Colla* liquid are mixed to obtain the Liang Di Tang water-extracted liquid. A mass ratio of the wine-processed *Rehmanniae Radix,* the *Scrophulariae Radix,* the wine-processed *Paeoniae Radix Alba,* the *Ophiopogon japonicus,* the *Lycii Cortex,* and *Asini Corii Colla* is 37.30 : 37.30 : 18.65 : 18.65 : 11.19 : 11.19. In some embodiments of the present disclosure, the *Asini Corii Colla* liquid is prepared by a process including the following steps: placing the *Asini Corii Colla* in water and liquefying the *Asini Corii Colla,* and sieving to obtain an undersized part, namely the *Asini Corii Colla* liquid. In some embodiments of the present disclosure, a mass of the water is 2 times to 6 times, preferably 3 times to 5 times, and further more preferably 4 times a mass of the *Asini Corii Colla.* In some embodiments of the present disclosure, the dissolution is conducted in a boiling water bath under stirring. In the present disclosure, there is no special limitation on a speed and time of the stirring, as long as the *Asini Corii Colla* could be liquefied to produce a homogeneous *Asini Corii Colla* liquid. In some embodiments, after the *Asini Corii Colla* is liquefied, the method further includes: removing foams and impurities in an upper layer of an *Asini Corii* Colla-liquefied system, and then sieving. In some embodiments of the present disclosure, a sieve used for the sieving has a sieve size of 100 mesh. In the present disclosure, there is no special limitation on the mixing, as long as the raw materials could be thoroughly mixed.

The present disclosure provides a Liang Di Tang water-extracted liquid prepared by the method as described in the above technical solutions. In some embodiments of the present disclosure, the Liang Di Tang water-extracted paste has a density of 1.2 g/mL to 1.3 g/mL and preferably 1.25 g/mL.

The present disclosure provides a Liang Di Tang water-extracted paste produced by concentrating the Liang Di Tang water-extracted liquid as described in the above technical solutions. In the present disclosure, the Liang Di Tang water-extracted paste has a density of 1.2 g/mL to 1.3 g/mL, preferably 1.22 g/mL to 1.28 g/mL, and more preferably 1.25 g/mL. In some embodiments of the present disclosure, conditions for the concentrating are the same as the conditions for the concentrating in the preparation process of the first concentrate, which are not repeated here. In the present disclosure, there is no special limitation on a time of the concentrating, as long as the concentrating could be conducted to a density of 1.2 g/mL to 1.3 g/mL.

The present disclosure provides a Liang Di Tang preparation prepared from raw materials including a Liang Di Tang water-extracted extract and a pharmaceutically acceptable adjuvant; the Liang Di Tang water-extracted extract is the Liang Di Tang water-extracted liquid as described in the above technical solutions and/or the Liang Di Tang water-extracted paste as described in the above technical solutions; a mass ratio of the pharmaceutically acceptable adjuvant to the Liang Di Tang water-extracted extract is in a rang of 1 : (12.48-18.72), preferably 1 : (13-18), more preferably 1 : (14-17), and further more preferably 1 : (15-16); and a mass of the Liang Di Tang water-extracted extract is based on a total mass of wine-processed *Rehmanniae Radix, Scrophulariae Radix,* wine-processed *Paeoniae Radix Alba, Ophiopogon japonicus, Lycii Cortex,* and *Asini Corii Colla.* In some embodiments, the pharmaceutically acceptable adjuvant includes one or more selected from the group consisting of dextrin, starch, and lactose.

In some embodiments of the present disclosure, in parts by mass, raw materials for preparing 1,000 parts of the Liang Di Tang preparation include: in parts by mass, raw materials for preparing 1,000 parts of the Liang Di Tang preparation include: 468 parts to 572 parts (more preferably 480 parts to 550 parts and further more preferably 500 parts to 520 parts) of the wine-processed *Rehmanniae Radix*; 468 parts to 572 parts (more preferably 480 parts to 550 parts and further more preferably 500 parts to 520 parts) of the *Scrophulariae Radix*; 234 parts to 286 parts (more preferably 240 parts to 270 parts and further more preferably 250 parts to 260 parts) of the wine-processed *Paeoniae Radix Alba;* 234 parts to 286 parts (more preferably 240 parts to 270 parts and further more preferably 250 parts to 260 parts) of the *Ophiopogon japonicus;* 140 parts to 171 parts (more preferably 150 parts to 170 parts and further more preferably 156 parts to 165 parts) of the *Lycii Cortex*; 140 parts to 171 parts (more preferably 150 parts to 170 parts and further more preferably 156 parts to 165 parts) of the *Asini Corii Colla;* and 100 parts to 150 parts (more preferably 110 parts to 140 parts and further more preferably 120 parts to 130 parts) of the pharmaceutically acceptable adjuvant. In some embodiments of the present disclosure, the Liang Di Tang preparation has a water content of 3 wt% to 8 wt%, more preferably 4 wt% to 7 wt%, and further more preferably 5 wt% to 6 wt%. In some embodiments of the present disclosure, the Liang Di Tang preparation has a dry extract yield of 40.0% to 55.0% and preferably 45% to 50%.

In some embodiments of the present disclosure, the Liang Di Tang preparation includes a Liang Di Tang powder or a Liang Di Tang granule. In some embodiments, the Liang Di Tang powder has a particle size of 150 µm to 850 µm; the Liang Di Tang granule preferably has a particle size of 180 µm to 2,000 µm.

The present disclosure provides a method for preparing the Liang Di Tang preparation as described in the above technical solutions, including the following steps: mixing and concentrating the Liang Di Tang water-extracted extract and an aqueous solution of the pharmaceutically acceptable adjuvant to obtain a mixed concentrate; and subjecting the mixed concentrate to vacuum belt drying to obtain the Liang Di Tang preparation (namely, the Liang Di Tang powder).

In some embodiments of the present disclosure, the aqueous solution of the pharmaceutically acceptable adjuvant is obtained by dissolving the pharmaceutically acceptable adjuvant in water. In some embodiments, a mass ratio of the pharmaceutically acceptable adjuvant to the water is in a range of 1 : (2-6), preferably 1 : (3-5), and further more preferably 1 : 4. In some embodiments, the dissolving is conducted under stirring. In the present disclosure, there is no special limitation on a speed and time of the stirring, as long as a homogeneous aqueous solution of the pharmaceutically acceptable adjuvant could be obtained.

In some embodiments of the present disclosure, conditions for the mixing and concentrating are the same as the conditions for the concentrating in the preparation process of the first concentrate, which are not repeated here. In the present disclosure, there is no special limitation on a time of the mixing and concentrating, as long as the mixed concentrate could have a density of 1.25 g/mL to 1.36 g/mL. In some embodiments, the mixed concentrate has a density of 1.28 g/mL to 1.34 g/mL and preferably 1.30 g/mL to 1.32 g/mL.

In some embodiments of the present disclosure, the vacuum belt drying is conducted in a vacuum belt dryer. In some embodiments, parameters for the vacuum belt drying include: a feeding temperature of 75 °C to 85 °C and preferably 80 °C; a feeding rate of 6 L/h to 24 L/h and preferably 10 L/h to 20 L/h; a running speed of a track being 16±5 cm/min and preferably 16±2 cm/min; an angle of a swingarm of 65° to 85° and preferably 70° to 80°; a speed of a material-distributing motor being 30 r/min to 50 r/min and preferably 40 r/min; heating temperatures in a first zone, a second zone, a third zone, and a fourth zone being 95±5 °C (preferably 95±2 °C), 96±5 °C (preferably 96±2 °C), 96±5 °C (preferably 96±2 °C), and 30±5 °C (preferably 30±2 °C, cooling), respectively; a speed of a cutter of 10 s/time to 20 s/time and preferably 15 s/time; and a vacuum degree of 0.097 MP to 0.1 MP and preferably 0.098 MPa to 0.099 MPa.

In some embodiments of the present disclosure, under the condition that the Liang Di Tang preparation is the Liang Di Tang granule, the method further includes granulation after the vacuum belt drying to obtain the Liang Di Tang granule. In some embodiments of the present disclosure, the granulation is conducted using a granulator. In some embodiments, parameters for the granulation include: a pressure of a press roller being 12 MPa to 20 MPa and more preferably 15 MPa to 18 MPa; a vertical rotational speed of a screw feeder being 18 r/min to 30 r/min and preferably 20 r/min to 25 r/min; a horizontal rotational speed of the screw feeder being 90 r/min to 140 r/min and preferably 100 r/min to 120 r/min; a rotational speed of the press roller being 4 r/min to 10 r/min and preferably 5 r/min to 8 r/min; and a granule sizing speed being 100 r/min to 150 r/min and preferably 120 r/min to 130 r/min. In a specific embodiment of the present disclosure, during the granulation, conditions of the granulator (for example, whether the powder discharge of the granulator is smooth, whether the running pressure is stable, and whether the granule discharge is smooth) and the appearance of particles (such as whether particles are uniform) are observed. The parameters of the granulator (such as the pressure of the press roller and the feeding rate) are adjusted according to the running conditions of the granulator and the appearance of particles, and the powder is fed and the particles are collected in time. The adjustment for the granulation parameters is recorded. A power supply for a vibrating sieve is turned on. In some embodiments, particles produced after the granulation are placed in a hopper, and a valve of the hopper is adjusted, such that a feeding rate allows the complete separation of qualified particles (a particle size of 180 µm to 2,000 µm) from a fine powder; the qualified particles (the Liang Di Tang granule) are placed in a transfer barrel, and the fine powder is re-granulated.

The present disclosure also provides a quality control standard for the Liang Di Tang preparation as described in the above technical solutions or the Liang Di Tang preparation prepared by the method as described in the above technical solutions, including an HPLC characteristic chromatogram, characteristic component contents, a water content, and a dry extract yield of the Liang Di Tang preparation;
the characteristic component contents include: a harpagoside content of 0.33 mg/g to 0.67 mg/g, a paeoniflorin content of 2.62 mg/g to 4.96 mg/g, an L-hydroxyproline content of 8.83 mg/g to 16.42 mg/g, and a glycine content of 17.42 mg/g to 32.46 mg/g;
the water content ranges from 3 wt% to 8 wt%; and
the dry extract yield ranges from 40% to 55%.

In the present disclosure, the harpagoside content is a mass content index for *Scrophulariae Radix,* the paeoniflorin content is a mass content index for wine-processed *Paeoniae Radix Alba,* and the L-hydroxyproline and glycine contents are mass content indexes for *Asini Corii Colla.*

In some embodiments of the present disclosure, the HPLC characteristic chromatogram of the Liang Di Tang preparation is acquired by a process including the following steps:
(1) mixing and concentrating the Liang Di Tang preparation to be tested, diatomaceous earth, and an aqueous acetonitrile solution to obtain a concentrate, and dissolving the concentrate in an aqueous methanol solution to obtain a test sample solution;
(2) mixing a paeoniflorin reference substance, a harpagoside reference substance, and methanol to obtain a reference substance solution; and
(3) subjecting the test sample solution and the reference substance solution to HPLC test separately to obtain a reference substance characteristic chromatogram and a test sample characteristic chromatogram; and based on a retention time of the paeoniflorin reference substance in the reference substance characteristic chromatogram, calculating a relative retention time of each of other characteristic peaks in the test sample characteristic chromatogram to obtain the HPLC characteristic chromatogram of the Liang Di Tang preparation,
where step (1) and step (2) are conducted in any order

In the present disclosure, the Liang Di Tang preparation to be tested, diatomaceous earth, and the aqueous acetonitrile solution are mixed and then concentrated to produce the concentrate, and the concentrate is dissolved in the aqueous methanol solution to produce the test sample solution. In some embodiments of the present disclosure, a concentration of the Liang Di Tang preparation to be tested in the test sample solution is in a range of 0.03 g/mL to 0.10 g/mL and preferably 0.05 g/mL to 0.08 g/mL. In some embodiments of the present disclosure, a mass ratio of the Liang Di Tang preparation to the diatomaceous earth is in a range of 1 : (0.5-1.5) and preferably 1 : 1. In some embodiments of the present disclosure, a volume fraction of acetonitrile in the aqueous acetonitrile solution is in a range of 70% to 90% and preferably 80%. In some embodiments of the present disclosure, a volume fraction of methanol in the aqueous methanol solution is in a range of 5% to 15% and preferably 10%. In a specific embodiment of the present disclosure, the test sample solution is prepared by a process including the following steps: grinding the Liang Di Tang preparation to be tested to fine, accurately weighing 1 g of a powder of the Liang Di Tang preparation to be tested and placing in a conical flask with a stopper, adding 1 g of the diatomaceous earth thereto, and mixing to be uniform, and accurately adding 25 mL of an 80% aqueous acetonitrile solution (a volume fraction of acetonitrile is 80%) thereto; weighing a weight of a resulting system, sonicating for 20 min at room temperature, 800 W, and 40 kHz, cooling, and weighing the resulting system; supplementing 80% aqueous acetonitrile solution to make up for a weight loss; shaking the conical flask to be uniform, and filtering; accurately taking 15 mL of a resulting filtrate and subjecting the filtrate to evaporation to dryness to produce a residue; dissolving the residue with a 10% aqueous methanol solution and then transferring a resulting solution to a 10 mL volumetric flask; adding the 10% aqueous methanol solution to the volumetric flask to a marked volume; shaking to be uniform, and then filtering through a microporous filter membrane to obtain a filtrate, i.e., the test sample solution.

In the present disclosure, the paeoniflorin reference substance, the harpagoside reference substance, and methanol are mixed to obtain a reference substance solution. In some embodiments of the present disclosure, a concentration of the paeoniflorin reference substance in the reference substance solution is in a range of 180 µg/mL to 200 µg/mL, and a concentration of the harpagoside reference substance in the reference substance solution is preferably 10 µg/mL to 30 µg/mL and more preferably 20 µg/mL.

In the present disclosure, after the test sample solution and the reference substance solution are obtained, the test sample solution and the reference substance solution each are subjected to HPLC test to obtain contrasted characteristic chromatograms. Based on the retention time of the chromatographic peak of the reference substance, the relative retention time of each of other common peaks is calculated to obtain the HPLC characteristic chromatogram of the Liang Di Tang preparation.

In some embodiments of the present disclosure, conditions for the HPLC include: adopting a chromatographic column with octadecylsilane-bonded silica gel as a filler; a mobile phase A being methanol, a mobile phase B being a 0.05 v/v% phosphoric acid solution, and a mobile phase flow rate ranging from 0.8 mL/min to 1.2 mL/min and preferably being 1 mL/min; an elution mode of gradient elution, and a program for the gradient elution being shown in Table 1; a detection wavelength of 254 nm; a column temperature of 28 °C; and an injection sample volume of 10 µL. In some embodiments, the number of theoretical plates should not be less than 2,000 based on a peak of paeoniflorin.

**Table 1 Gradient elution program**

| Time (min) | Volume fraction of the mobile phase A (%) | Volume fraction of the mobile phase B (%) |
|---|---|---|
| 0-10 | 5→23.5 | 95→76.5 |
| 10-20 | 23.5 | 76.5 |
| 20-58 | 23.5→63 | 76.5→37 |
| 58-60 | 63→90 | 37→10 |

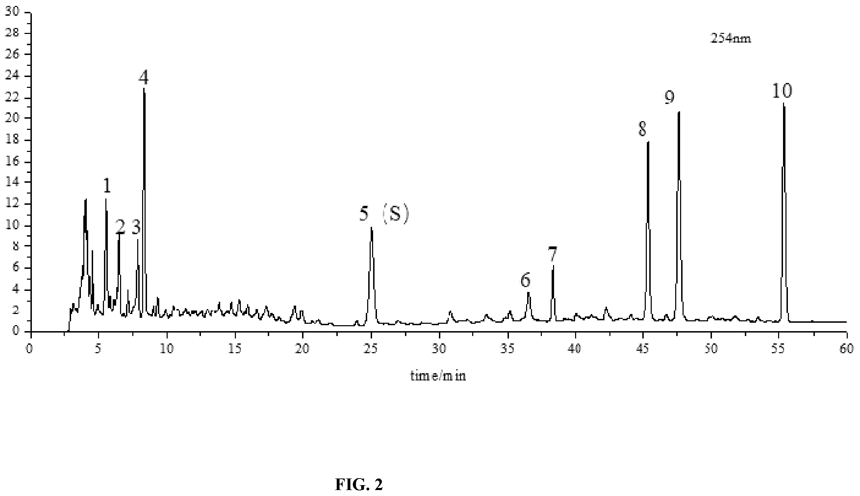

In the present disclosure, the contrasted characteristic chromatograms are shown in FIG. 2. 10 characteristic peaks are present in a test sample characteristic chromatogram, 2 peaks of which should each have a respective retention time consistent with a peak of a corresponding reference substance (the paeoniflorin reference substance and the harpagoside reference substance). A peak corresponding to the paeoniflorin reference substance is taken as an S peak. A relative retention time of each characteristic peak relative to the S peak is calculated, and the relative retention time should be within ±10% of a specified value. In some embodiments, the specified value is as follows: 0.22 (peak 1), 0.26 (peak 2), 0.31 (peak 3), 0.33 (peak 4), 1.00 (peak 5), 1.47 (peak 6), 1.54 (peak 7), 1.82 (peak 8), 1.91 (peak 9), and 2.22 (peak 10). The peaks 1 to 3 are attributed to unknown components. The peak 4 is attributed to gallic acid. The peak 5 is attributed to paeoniflorin. The peak 7 is attributed to verbascoside. The peak 8 is attributed to angoroside C. The peak 9 is attributed to cinnamic acid. The peak 10 is attributed to harpagoside.

In some embodiments of the present disclosure, a dry extract yield is tested according to the General Rule 0104 in the *Chinese Pharmacopoeia,* 2020 edition.

A Liang Di Tang preparation to be tested is finely ground to produce a powder, 4 g of the powder is taken, added to 30 mL of methanol, and a resulting mixture is subjected to ultrasonic treatment for 30 min, and filtered to obtain a filtrate, and the filtrate is concentrated to 5 mL to obtain a test sample solution. 1.5 g of a *Lycii Cortex* as a control medicine is taken, added to 30 mL of methanol, and a resulting mixture is subjected to ultrasonic treatment for 30 min, and filtered to produce a filtrate, and the filtrate is concentrated to 5 mL to obtain a control medicine solution. 10 µL of each of the test sample solution and the control medicine solution is pipetted and spotted on a same silica gel G thin-layer chromatography plate. With cyclohexane-ethyl acetate-formic acid (at a volume ratio of 5 : 5 : 0.4) as a developing solvent, developing is conducted. The silica gel G thin-layer chromatography plate is taken out, air-dried, and inspected under an ultraviolet lamp (365 nm). Fluorescent spots in a same color appear at a position in the chromatography plate of the test sample that corresponds to the chromatography plate of the control medicine, indicating *Lycii Cortex.*

In the present disclosure, there is no special limitation on a test method for the water content, and a water content test method well known to those skilled in the art may be adopted, specifically such as an oven-drying method.

In the present disclosure, test methods for contents of paeoniflorin and harpagoside are the same as the method for acquiring the HPLC characteristic chromatogram of the Liang Di Tang preparation and the method for acquiring the HPLC characteristic chromatogram of the Liang Di Tang preparation, which will not be repeated here.

In some embodiments of the present disclosure, a test method for contents of L-hydroxyproline and glycine includes the following steps:
(1) A Liang Di Tang preparation to be tested is finely ground to produce a powder. 1.5 g of the powder is added to a 25 mL volumetric flask, 20 mL of a 0.1 mol/L aqueous hydrochloric acid solution is added, and a resulting mixture is subjected to ultrasonic treatment for 30 min, and cooled. A 0.1 mol/L aqueous hydrochloric acid solution is added to a marked volume, and the volumetric flask is thoroughly shaken to obtain a solution of the Liang Di Tang preparation in hydrochloric acid. 2 mL of the solution of the Liang Di Tang preparation in hydrochloric acid is added to a 10 mL ampoule, 2 mL of 0.08 mol/L to 0.12 mol/L hydrochloric acid is added thereto, and a resulting mixture is subjected to hydrolysis at 150 °C for 1 h to obtain a hydrolysis system. The hydrolysis system is cooled, transferred to an evaporation dish, and washed in batches with 10 mL of water. Resulting washing solutions are combined in an evaporation dish and subjected to evaporation to dryness to produce a residue. The residue is dissolved with a 0.1 mol/L aqueous hydrochloric acid solution and transferred to a 25 mL volumetric flask, a 0.1 mol/L aqueous hydrochloric acid solution is added to a marked volume, and the volumetric flask is thoroughly shaken to obtain a test sample solution.
(2) An L-hydroxyproline reference substance, a glycine reference substance, and an aqueous hydrochloric acid solution are mixed to produce a reference substance solution. In the reference substance solution, a concentration of L-hydroxyproline is 60 µg/mL and a concentration of glycine is 130 µg/mL.
(3) 1 mL of the test sample solution is accurately taken and added to a 25 mL test tube with a stopper, and 0.5 mL of a solution of 0.1 mol/L phenyl isothiocyanate (PITC) in acetonitrile and 0.5 mL of a solution of 1 mol/L triethylamine in acetonitrile are added thereto. The test tube is thoroughly shaken and placed at room temperature for 1 h. 3 mL of a 50% (v/v) aqueous acetonitrile solution is added, and the test tube is thoroughly shaken. 5 mL of n-hexane is added, and the test tube is shaken and placed for 10 min. A solution in a lower layer is collected and filtered through a microporous filter membrane to produce a filtrate, which is a derived test sample solution.
(4) In step (3), the test sample solution is replaced with the reference substance solution to obtain a derived reference substance solution.
(5) The derived test sample solution and the derived reference substance solution each are subjected to HPLC test to obtain the contents of L-hydroxyproline and glycin;
Step (1) and step (2) are conducted in any order; and step (3) and step (4) are conducted in any order.

In some embodiments of the present disclosure, conditions for the HPLC include: adopting a chromatographic column with octadecylsilane-bonded silica gel as a filler; a mobile phase A being acetonitrile-0.1 mol/L sodium acetate solution (a pH is adjusted with acetic acid to 6.5) (7 : 93), and a mobile phase B being acetonitrile-water (a volume ratio being 4 : 1); an elution mode of gradient elution, and a program for the gradient elution is shown in Table 2; a mobile phase flow rate being 0.8 mL/min; a detection wavelength of 254 nm; a column temperature of 43 °C; and an injection sample volume of 5 µL. In some embodiments, the number of theoretical plates is calculated to be 4,000 to 10,000 according to a peak of L-hydroxyproline.

**Table 2 Gradient elution program**

| Time (min) | Volume fraction of the mobile phase A (%) | Volume fraction of the mobile phase B (%) |
|---|---|---|
| 0-11 | 100→93 | 0→7 |
| 11-13.9 | 93→88 | 7→12 |
| 13.9-14 | 88→85 | 12→15 |
| 14-29 | 85→66 | 15→34 |
| 29-30 | 66→0 | 34→100 |

The technical solutions of the present disclosure will be clearly and completely described below in conjunction with examples of the present disclosure. Apparently, the described examples are merely some rather than all of the examples of the present disclosure. All other examples obtained by those of ordinary skill in the art based on the examples of the present disclosure without creative efforts shall fall within the scope of the present disclosure.

In the following examples of the present disclosure, each traditional Chinese medicine is processed before use. The processing requirements for each medicine in Liang Di Tang are determined based on Research on Materia Medica. A processing technology is studied through a test, and the detailed processing parameters such as soaking, cutting, drying, and an adjuvant amount are determined. Processing methods for the traditional Chinese medicines are as follows:

Wine-processed *Rehmanniae Radix*: (1) selection: removing impurities; (2) spray-washing: spray-washing for 10 min to 15 min; (3) enclosed moistening: enclosed moistening at a temperature of 15 °C to 35 °C for 8 h to 12 h; (4) cutting: with a gap between a blade and an edge being set to 3 mm, cutting into thick slices of 2 mm to 4 mm; (5) drying: with a layer thickness of 2 cm to 3 cm, drying at a temperature of 60 °C to 70 °C for 6 h to 7 h; (6) wine-frying: adding 15% yellow rice wine, and frying at 220 °C for 24 min until a surface temperature of the medicine is 130 °C to 135 °C; and (7) refined selection: sieving with a 3-mm medicinal sieve to remove debris, and manually discarding unqualified slices that are charred or do not have a required thickness.

*Scrophulariae Radix*: (1) selection: removing impurities; (2) spray-washing: spray-washing for 15 min to 20 min; (3) enclosed moistening: enclosed moistening at a temperature of 5 °C to 35 °C for 4 h to 26 h; (4) cutting: with a gap between a blade and an edge being set to 1.5 mm, cutting into thin slices of 1 mm to 2 mm; (5) drying: with a layer thickness of 2 cm to 3 cm, drying at a temperature of 50 °C to 60 °C for 3.5 h to 4.5 h; and (6) refined selection: sieving with a 2-mm medicinal sieve to remove debris, and manually discarding unqualified slices that are charred or do not have a required thickness.

Wine-processed *Paeoniae Radix Alba:* (1) selection: removing impurity and grading (small grade: a diameter of a root tip is smaller than 1.0 cm; and large grade: a diameter of a root tip is larger than or equal to 1.0 cm); (2) spray-washing: spray-washing for 10 min to 15 min; (3) soaking: at a temperature of 15 °C to 35 °C, soaking the medicine at the small grade for 12 h to 40 h, and soaking the medicine at the large grade for 24 h to 56 h; (4) cutting: with a gap between a blade and an edge being set to 1.5 mm, cutting into thick slices of 1 mm to 2 mm; (5) drying: with a layer thickness of 2 cm to 3 cm, drying at a temperature of 70 °C to 80 °C for 4 h to 5 h; (6) wine-frying: adding 20% yellow rice wine, and frying at 230 °C for 20 min until a surface temperature of the medicine is 140 °C to 145 °C; and (7) refined selection: sieving with a 3-mm medicinal sieve to remove debris, and manually discarding unqualified slices that are charred or do not have a required thickness.

*Ophiopogon japonicus:* (1) selection: removing impurities; (2) spray-washing: spray-washing for 10 min to 15 min; (3) enclosed moistening: enclosed moistening at a temperature of 5 °C to 35 °C for 6 h to 24 h; (4) flattening: with a roller clearance of 2 mm to 3 mm, pressing the medicine into flattened pieces; (5) drying: with a layer thickness of 2 cm to 3 cm, drying at a temperature of 50 °C to 60 °C for 6 h to 7 h; and (6) refined selection: manually discarding unqualified products that are not flattened.

*Lycii Cortex*: (1) selection: removing impurities and residual woody cores; (2) water-washing: washing with water until there is a natural color and there are free of dust impurities; (3) drying: with a layer thickness of 2 cm to 3 cm, drying at a temperature of 40 °C to 50 °C for 4 h to 5 h; (4) sieving: sieving with a 3-mm medicinal sieve to remove debris.

In the following examples, a preparation method of an accompanying decoction was as follows: 37.3 g of wine-processed *Rehmanniae Radix,* 37.3 g of *Scrophulariae Radix,* 18.6 g of wine-processed *Paeoniae Radix Alba,* 18.6 g of *Ophiopogon japonicus,* and 11.2 g of *Lycii Cortex* (five traditional Chinese medicines) were soaked in water for 40 min, water of a mass 7 times a mass of the five traditional Chinese medicines was added, and a resulting mixture was then decocted for 30 min, followed by filtration to obtain a first extraction solution and a first medicinal residue. Water of a mass 5 times the mass of the five traditional Chinese medicines was added to the first medicinal residue, and a resulting mixture was decocted for 20 min, followed by filtration to obtain a second extraction solution. The first extraction solution and the second extraction solution were combined and concentrated to about 500 mL. 11.2 of *Asini Corii Colla* is added and Yanghua (being melted by gentle heating). Vacuum concentration was conducted to about 250 mL, and lyophilization is conducted to constant weight to obtain a lyophilized powder, which was the accompanying decoction.

### Example 1

### Textual research on the Liang Di Tang formula and dosage

Liang Di Tang originates from the *Fu Qing-Zhu's Gynecology* written by the doctor Fu Shan (Qing Zhu) in the late Ming and early Qing dynasties. It is prescribed in the *Fu Qing-Zhu's Gynecology* that Liang Di Tang (daily dose) is composed of Da Sheng Di (1 Liang, wine-fried), Yuan Sheng (1 Liang), Bai Shao (Yao) (5 Qian, wine-fried), Mai Dong Rou (5 Qian), Di Gu Pi (3 Qian), and Ejiao (3 Qian).

Each medicine was subjected to Research on Materia Medica. Because the Liang Di Tang formula was proposed by the doctor Fu Shan in the late Ming and early Qing dynasties; in this study, the relevant descriptions for the medicines in the herbal references since the Ming and Qing dynasties were mainly verified and compared with the *Chinese Pharmacopoeia* of 2020 edition, the *Chinese Materia Medica,* and related records to determine the original plant varieties and processing methods for the medicines. The Da Sheng Di (wine-fried) was wine-processed *Rehmanniae Radix,* the Yuan Sheng was *Scrophulariae Radix,* the Bai Shao (Yao) (wine-fried) was wine-processed *Paeoniae Radix Alba,* the Mai Dong Rou was *Ophiopogon japonicus,* the Di Gu Pi was *Lycii Cortex,* and the Ejiao was *Asini Corii Colla.*

Through the textual research on the weights and measures in the Ming and Qing dynasties, the conversion relationships between the weights and measures in the Ming and Qing dynasties and the modern weights and measures were determined. In the Ming and Qing dynasties, a volume was measured in Sheng, Dou, and Hu, where 1 Hu = 5 Dou and 1 Dou = 10 Sheng. 1 Sheng is equivalent to 1,035 mL today. In the Ming and Qing dynasties, a weight was measured by Liang and Qian, where 1 Liang = 10 Qian. 1 Qian is equivalent to 3.73 g today. Therefore, the Liang Di Tang formula (daily dose) was converted as follows: 37.30 g of wine-processed *Rehmanniae Radix,* 37.30 g of *Scrophulariae Radix,* 18.65 g of wine-processed *Paeoniae Radix Alba,* 18.65 g of *Ophiopogon japonicus,* 11.19 g of *Lycii Cortex,* and 11.19 g of *Asini Corii Colla.*

### Example 2

### Preparation and quality standard for a Liang Di Tang reference sample

The "decocting in water for oral administration" for Liang Di Tang was originally stated without a detailed decoction technique. In the present disclosure, with reference to the "Management Standards for Traditional Chinese medicine Decoction Rooms in Medical Institutions" and the ancient decoction practices, the key process parameters including a soaking time, a water amount, a decoction power, a decoction time, and a concentration power were investigated and determined to establish the preparation process and quality standard for the Liang Di Tang reference sample.

### 1. Preparation of the Liang Di Tang reference sample

37.30 g of wine-processed *Rehmanniae Radix,* 37.30 g of *Scrophulariae Radix,* 18.65 g of wine-processed *Paeoniae Radix Alba,* 18.65 g of *Ophiopogon japonicus,* and 11.19 g of *Lycii Cortex* were placed in a 2 L decoction pot, and decocted twice with the decoction pot covered. Water was added at an amount 7 times a total amount of the five traditional Chinese medicines, and soaking was conducted for 40 min. Heating was conducted until boiling (power: 2,200 W, 7 min), and a slight boiling state (power: 400 W) was kept for 30 min (during which stirring was conducted once every 10 min, and the stirring was conducted for not less than 10 s each time). Filtration was conducted (200-mesh medicinal sieve) while hot to obtain a medicinal residue and a first decoction solution for later use. Water was then added at an amount 5 times the total amount of the five traditional Chinese medicines to the medicinal residue, a resulting mixture was heated until boiling (power: 2,200 W, about 5 min), and then a slight boiling state (power: 400 W) was kept for 20 min (during which stirring was conducted once every 10 min, and the stirring was conducted for not less than 10 s each time). Filtration was conducted (200-mesh medicinal sieve) while hot to obtain a second decoction solution for later use. The first decoction solution and the second decoction solution were combined in a 2 L decoction pot, concentrated under atmospheric pressure with the decoction pot uncovered (power: 1,200 W) to about 500 mL (concentration time: about 20 min). 11.19 g of *Asini Corii Colla* was added while hot and a resulting mixture was stirred and subjected to Yanghua (being melted by gentle heating) to obtain a Liang Di Tang reference sample solution (which had a density of 1.05±0.01 g/mL at 20 °C). The Liang Di Tang reference sample solution was vacuum-concentrated (T ≤ 60 °C) to about 250 mL (concentration time: about 1 h) to obtain a Liang Di Tang reference sample concentrate (which had a density of 1.07±0.01 g/mL at 20 °C). The Liang Di Tang reference sample concentrate was lyophilized (T ≤ -40 °C, and Vac ≤ 20 Pa) for 48 h to obtain the Liang Di Tang reference sample (a lyophilized powder with a water content of 4 wt% to 7 wt%). The Liang Di Tang reference sample was packaged with a ziplock bag in a sealed state and then stored in a dryer at room temperature.

### 2. Quality standard for the Liang Di Tang reference sample

2.1 Formula (daily dose): 37.30 g of wine-processed *Rehmanniae Radix,* 37.30 g of *Scrophulariae Radix,* 18.65 g of wine-processed *Paeoniae Radix Alba,* 18.65 g of *Ophiopogon japonicus,* 11.19 g of *Lycii Cortex,* and 11.19 g of *Asini Corii Colla.*

Characteristics: This product was a brownish-yellow to brownish powder with a sweet and slightly-bitter taste.

### 2.2 Characteristic chromatograms

Preparation of a reference substance solution: Appropriate amounts of a paeoniflorin reference substance and a harpagoside reference substance were weighed accurately, dissolved in methanol, and diluted to a specified volume to obtain the reference substance solution. In the reference substance solution, a concentration of the paeoniflorin reference substance was 200 µg/mL, and a concentration of the harpagoside reference substance was 30 µg/mL.

Preparation of a test sample solution: The Liang Di Tang reference sample prepared in step (1) was taken and finely ground to obtain a Liang Di Tang reference sample powder. 0.5 g of the Liang Di Tang reference sample powder was accurately weighed and added to an Erlenmeyer flask with a stopper, and 5 mL of water was accurately added for dissolution. 20 mL of acetonitrile was then accurately added to the Erlenmeyer flask under shaking, and then the Erlenmeyer flask was stoppered and weighed. An ultrasonic treatment was conducted for 20 min (with a frequency of 40 kHz and a power of 800 W), and a resulting system was naturally cooled (20 °C). The Erlenmeyer flask was weighed once again, and an 80% (v/v) aqueous acetonitrile solution was supplemented to make up for a weight loss. A resulting mixture was filtered, and 20 mL of a resulting filtrate was accurately collected and subjected to evaporation to dryness to obtain a residue. The residue was dissolved with a 10% (v/v) aqueous methanol solution and diluted to 10 mL to obtain the test sample solution.

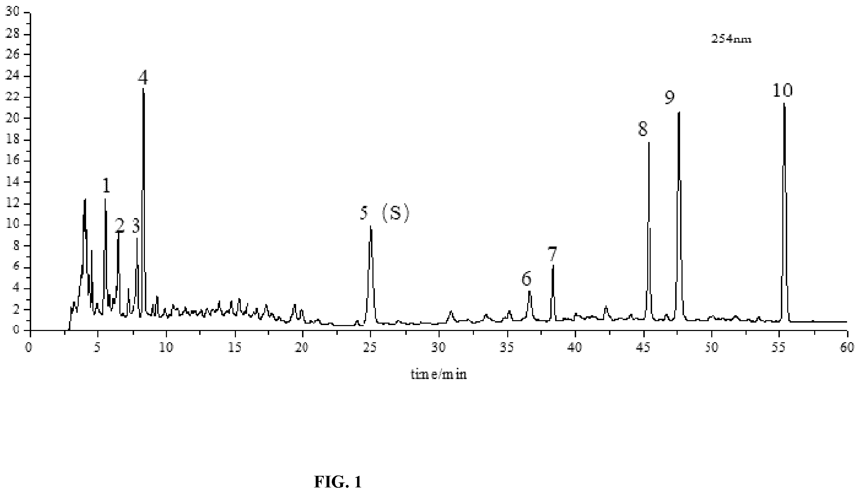

Determination: 10 µL of each of the reference substance solution and the test sample solution was accurately pipetted and injected into a high-performance liquid chromatograph for testing. Chromatogram within 60 min was recorded to obtain contrasted characteristic chromatogram (as shown in FIG. 1).

Chromatography conditions and system suitability testing: Octadecylsilane-bonded silica gel was adopted as a filler. Methanol was adopted as a mobile phase A and a 0.05 v/v% aqueous phosphoric acid solution was adopted as a mobile phase B. Gradient elution was conducted according to the gradient elution program shown in Table 1. A detection wavelength was 254 nm. A column temperature was 28 °C. The number of theoretical plates was calculated to be 20,000 to 30,000 based on a peak of paeoniflorin.

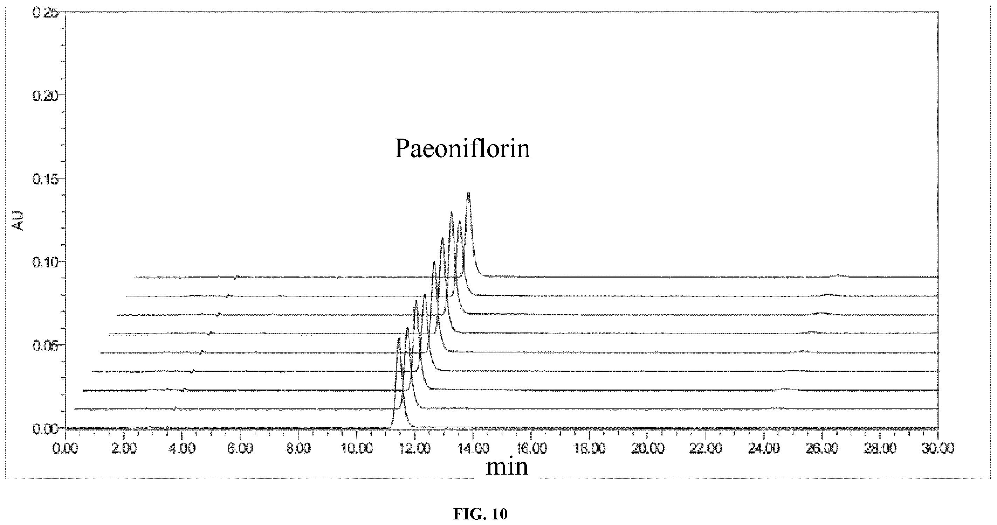

As shown in FIG. 1, 10 characteristic peaks are present in a test sample characteristic chromatogram, 2 peaks of which should each have a retention time consistent with a peak of a corresponding reference substance. A peak corresponding to the paeoniflorin reference substance is taken as an S peak. A relative retention time of each characteristic peak relative to the S peak is calculated, and the relative retention time should be within ±10% of a specified value. The specified value is as follows: 0.22 (peak 1), 0.26 (peak 2), 0.31 (peak 3), 0.33 (peak 4), 1.00 (peak 5), 1.47 (peak 6), 1.54 (peak 7), 1.82 (peak 8), 1.91 (peak 9), and 2.22 (peak 10). The peaks 1 to 3 are attributed to unknown components. The peak 4 is attributed to gallic acid. The peak 5 is attributed to paeoniflorin. The peak 7 is attributed to verbascoside. The peak 8 is attributed to angoroside C. The peak 9 is attributed to cinnamic acid. The peak 10 is attributed to harpagoside.

### 2.3 Identification, water content, and dry extract yield

Identification: (1) 4 g of the sample powder was taken and added to 30 mL of methanol. An ultrasonic treatment was conducted for 30 min (power: 400 W), and a resulting mixture was then filtered to obtain a filtrate. The filtrate was concentrated to 5 mL to obtain a test sample solution. 1.5 g of *Lycii Cortex* as a control medicine was taken to prepare a control medicine solution by the same method. 10 µL of each of the above two solutions was pipetted and spotted on a same silica gel G thin-layer chromatography plate. With a mixed solvent of cyclohexane, ethyl acetate, and formic acid at a volume ratio of 5 : 5 : 0.4 as a developing solvent, developing was conducted. The silica gel G thin-layer chromatography plate was taken out, air-dried, and inspected under an ultraviolet lamp (365 nm). Fluorescent spots in a same color appeared at a position in the chromatography plate of the test sample corresponding to the chromatography plate of the control medicine.

Detection: A water content ranged from 3 wt% to 8 wt%.

A dry extract yield ranged from 40.0% to 55.0%.

### 2.4 Determination of contents of harpagoside and paeoniflorin

Chromatography conditions and system suitability testing: A chromatographic column with octadecylsilane-bonded silica gel as a filler was adopted. Methanol was adopted as a mobile phase A and a 0.05 v/v% aqueous phosphoric acid solution was adopted as a mobile phase B. A gradient elution program was shown in Table 1. A detection wavelength was 254 nm. A column temperature was 28 °C. The number of theoretical plates was calculated to be 20,000 to 35,000 based on a peak of paeoniflorin.

Preparation of a reference substance solution: A paeoniflorin reference substance and a harpagoside reference substance were weighed accurately, dissolved in methanol, and diluted to a specified volume to obtain the reference substance solution in which a concentration of harpagoside was 20 µg/mL and a concentration of paeoniflorin was 180 µg/mL.

The test sample solution was prepared according to step 2.2.

Determination: 10 µL of each of the reference substance solution and the test sample solution was accurately pipetted, injected into a high-performance liquid chromatograph, and subjected to HPLC test to obtain the contents of harpagoside and paeoniflorin.

Each dose (daily dose) included 24 mg to 47 mg of *Scrophulariae Radix* in terms of harpagoside and 198 mg to 370 mg of wine-processed *Paeoniae Radix Alba* in terms of paeoniflorin.

### 2.5 Determination of contents of L-hydroxyproline and glycine

Chromatography conditions and system suitability testing: A chromatographic column with octadecylsilane-bonded silica gel as a filler was adopted. An acetonitrile-0.1 mol/L sodium acetate solution (a pH was adjusted with acetic acid to 6.5) (a volume ratio of the acetonitrile to the sodium acetate solution was 7 : 93) was adopted as a mobile phase A, and an 80% (v/v) aqueous acetonitrile solution was adopted as a mobile phase B. Gradient elution was conducted according to the gradient elution program in Table 2. A flow rate was 0.8 mL/min. A detection wavelength was 254 nm. A column temperature was 43 °C. The number of theoretical plates was calculated to be 56,000 to 10,000 based on a peak of L-hydroxyproline.

Preparation of a reference substance solution: Appropriate amounts of an L-hydroxyproline reference substance and a glycine reference substance were weighed accurately and dissolved with a 0.1 mol/L hydrochloric acid solution to prepare a mixed solution containing 60 µg of L-hydroxyproline and 130 µg of glycine per 1 mL.

Preparation of a test sample solution: 1.5 g of the sample powder was weighed accurately and added to a 25 mL volumetric flask, 20 mL of a 0.1 mol/L hydrochloric acid solution was added, and an ultrasonic treatment was conducted for 30 min. A resulting mixture was naturally cooled, a 0.1 mol/L hydrochloric acid solution was added thereto to the marked volume, and the volumetric flask was fully shaken to obtain a solution. 2 mL of the solution was accurately taken and added to a 10 mL ampoule, 2 mL of hydrochloric acid was then added thereto, and a resulting mixture was subjected to hydrolysis at 150 °C for 1 h. A resulting hydrolysis system was naturally cooled, transferred to an evaporation dish, and washed with 10 mL of water in batches. Resulting washing solutions were combined in an evaporation dish, and subjected to evaporation to dryness to obtain a residue. The residue was dissolved with a 0.1 mol/L hydrochloric acid solution, and a resulting solution was transferred to a 25 mL volumetric flask, and diluted with a 0.1 mol/L hydrochloric acid solution to the marked volume, followed by thoroughly shaking.

Derivation: 1 mL of each of the reference substance solution and the test sample solution was accurately taken and added separately to 25 mL test tubes each with a stopper, and 0.5 mL of a solution of 0.1 mol/L PITC in acetonitrile and 0.5 mL of a solution of 1 mol/L triethylamine in acetonitrile were added to each test tube. The test tubes were thoroughly shaken and placed at room temperature for 1 h. 3 mL of a 50% (v/v) aqueous acetonitrile solution was added to each test tube, and the test tubes were thoroughly shaken. 5 mL of n-hexane was added to each test tube, and the test tubes were shaken and placed for 10 min. Resulting solutions in lower layers were collected and filtered through a microporous filter membrane to obtain filtrates, which were a derived reference substance solution and a derived test sample solution, respectively.

Determination: 5 µL of each of the derived reference substance solution and the derived test sample solution was accurately pipetted, injected into a high-performance liquid chromatograph, and subjected to HPLC test, to obtain the contents of L-hydroxyproline and glycine.

Each dose of the Liang Di Tang product contained 0.64 g to 1.18 g of *Asini Corii Colla* in terms of L-hydroxyproline and 1.26 g to 2.33 g of *Asini Corii Colla* in terms of glycine.

2.6 Functions and indication: The Liang Di Tang product exhibits the function of nourishing yin and clearing heat and the function of nourishing blood and normalizing menstrual cycle. The Liang Di Tang product has the indication of Yin deficiency with blood-heat syndrome, which is characterized by the following symptoms: early menstruation, scanty and red menstrual flow, heat sensation in hands and feet, irritation and insomnia, red tongue with scanty coating, and thin and rapid pulse.

Precautions: The Liang Di Tang product should not be used for the early menstruation due to excessive heat and blood stasis, and should be used with caution in patients with spleen-stomach deficiency and loose stools.

Storage: The Liang Di Tang product should be stored with sealed in a dry environment.

### Example 3

### Research on a preparation process of a Liang Di Tang granule

Chinese herbal slices for Liang Di Tang: 37.30 g of wine-processed *Rehmanniae Radix,* 37.30 g of *Scrophulariae Radix,* 18.65 g of wine-processed *Paeoniae Radix Alba,* 18.65 g of *Ophiopogon japonicus,* and 11.19 g of *Lycii Cortex.*

### 1. Research on an extraction process

### 1.1 Investigation of a soaking time

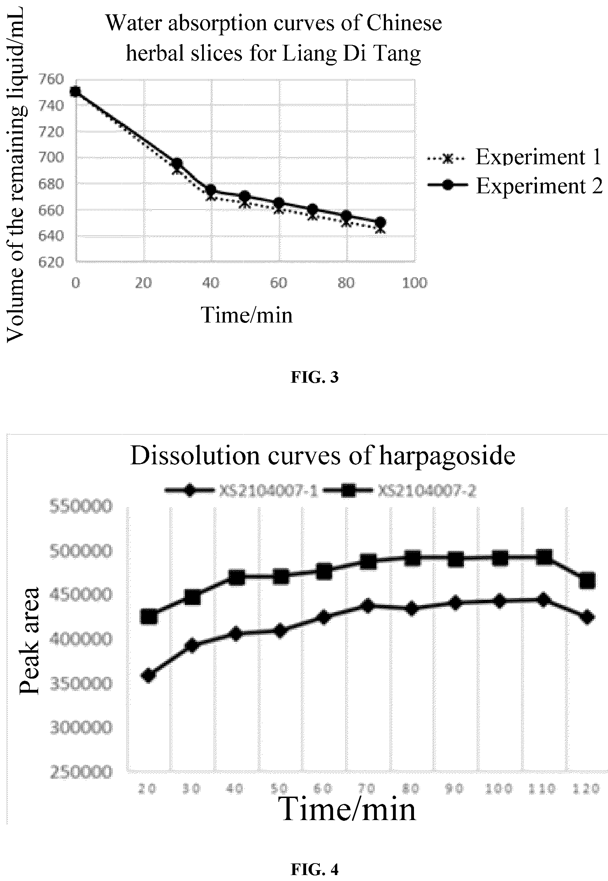

Seven groups of the Chinese herbal slices for Liang Di Tang were weighed and placed in seven 2 L Erlenmeyer flasks, respectively. 750 mL of water was added to each Erlenmeyer flask, and the Erlenmeyer flasks were stoppered. Soaking was conducted at room temperature for 0 min, 30 min, 40 min, 50 min, 60 min, 70 min, 80 min, and 90 min in respective Erlenmeyer flask. After the soaking was completed, a volume of the remaining liquid in each Erlenmeyer flask was measured and recorded as V. A water absorption curve was plotted. Two parallel experiments were conducted. Water absorption results of the Chinese herbal slices for Liang Di Tang are shown in Table 3 and FIG. 3.

It can be seen from Table 3 and FIG. 3 that the water absorption amount of the Chinese herbal slices for Liang Di Tang encounters an inflection point at 40 min. That is to say, the water absorption amount is large within the first 40 min, and decreases and tends to be stable after 40 min. Although the extension of the soaking time is conducive to the full impregnation of the Chinese herbal slices for Liang Di Tang and the dissolution of ingredients during decoction, a too-long soaking time is also easy to increase the risks of enzymatic hydrolysis and moldy deterioration of a medicinal liquid. According to comprehensive consideration, the soaking time for the Chinese herbal slices of Liang Di Tang before decoction is determined to be 40 min.

**Table 3 Investigation results of water absorption amount of the Chinese herbal slices for Liang Di Tang**

| Soaking time (min) | Volume of the remaining liquid, V/mL | |
|---|---|---|
| | Experiment 1 | Experiment 2 |
| 0 | 750 | 750 |
| 30 | 690 | 695 |
| 40 | 670 | 675 |
| 50 | 665 | 670 |
| 60 | 660 | 665 |
| 70 | 655 | 660 |
| 80 | 650 | 655 |
| 90 | 645 | 650 |

### 1.2 Investigation of the number of extractions and the extraction time

Test method (2 parallel tests were carried out for each test): The Chinese herbal slices for Liang Di Tang (wine-processed *Rehmanniae Radix*: Batch No. 20181210, *Scrophulariae Radix*: Batch No. 200501CP0184, wine-processed *Paeoniae Radix Alba:* Batch No. 191101CP159, *Ophiopogon japonicus:* Batch No. 201909-001, and *Lycii Cortex*: Batch No. 20190312) were weighed in a mass 1.5 times the above specified mass and added to a round-bottomed flask, and water was added thereto. Soaking was conducted at room temperature for 40 min. Heating was then conducted to allow reflux extraction 3 times (water amount and the extraction time for each extraction shown in Table 4). Resulting extraction solutions were combined and sieved through a 200-mesh sieve for later use. Investigation results of the number of extractions and the extraction time for the Liang Di Tang granule are shown in Table 5.

**Table 4 Conditions for investigation tests of the number of extractions and the extraction time for the Liang Di Tang granule**

| Batch No. | Number of extractions | Time of each extraction | Water amount for each extraction |
|---|---|---|---|
| LDTXS2102001-1 | 3 | 30 min, 20 min, and 20 min | 7 times, 5 times, and 5 times |
| LDTXS2102001-2 | | | |
| LDTXS2102002-1 | 3 | 90 min, 60 min, and 60 min | 7 times, 5 times, and 5 times |
| LDTXS2102002-2 | | | |
| Accompanying decoction | 2 | 30 min and 20 min | 7 times and 5 times |

**Table 5 Investigation results of the number of extractions and the extraction time for the Liang Di Tang granule**

| Batch No. | Dry extract yield/% | Harpagoside | | Paeoniflorin | |
|---|---|---|---|---|---|
| | | Content/wt% | Transfer rate/% | Content/wt% | Transfer rate/% |
| LDTXS2102001-1 | 55.92 | 0.102 | 100.99 | 2.093 | 93.86 |
| LDTXS2102001-2 | 55.42 | 0.097 | 96.04 | 1.940 | 87.00 |
| LDTXS2102002-1 | 58.55 | 0.075 | 74.26 | 1.754 | 78.65 |
| LDTXS2102002-2 | 58.08 | 0.086 | 85.15 | 1.838 | 82.42 |
| Accompanying decoction | 40.81 | 0.0547 | 54.16 | 1.341 | 60.13 |

| | | | | | |
|---|---|---|---|---|---|
| Notes: A content of harpagoside in *Scrophulariae Radix* (200501CP0184) is 0.101 wt%, and a content of paeoniflorin in wine-processed *Paeoniae Radix Alba* (191101CP0159) is 2.23 wt%. | | | | | |

Results in Table 5 show: (1) After the third extraction is added in the extraction process for the Liang Di Tang granule, the dry extract yield and the transfer rates of harpagoside and paeoniflorin as quality indexes increase by 14.86%, 44.16%, and 29.98%, respectively. (2) With the extension of the extraction time, the dry extract yield and the transfer rates of harpagoside and paeoniflorin increase by 12.31%, 19.72%, and 15.67%, respectively. (3) The increase of the number of extractions could significantly increase the extraction rate, and the extension of the extraction time could also increase the extraction rate; with the increase of the number of extractions, the dry extract yield increases, but the contents of harpagoside and paeoniflorin drop to some extent because harpagoside and paeoniflorin partially undergo thermal destruction with the extension of the decoction time. (4) According to the preliminary analysis, the extraction process including three extractions for the Liang Di Tang granule is preferred, and results in a high dry extract yield and high extraction rates for harpagoside and paeoniflorin.

### 1.3 Investigation for the extraction time and the water amount

Test method: The Chinese herbal slices for Liang Di Tang were weighed in a mass 80 times, 70 times, and 65 times the above specified mass and added to a 100 L extraction and concentration integrated machine, and water was added thereto. Soaking was conducted at room temperature for 40 min, and heat extraction was conducted at 99.5±0.5 °C (extraction conditions shown in Table 6). Test results for the extraction time and the water amount are shown in Tables 7 and 8.

**Table 6 Conditions for investigation tests of the number of extractions and the extraction time for the Liang Di Tang granule**

| Batch No. | Number of extractions | Time of each extraction | Water amount for each extraction |
|---|---|---|---|
| LDTXS2102003 | 3 | 30 min, 20 min, and 20 min | 7 times, 5 times, and 5 times |
| LDTXS2102004 | 4 | 45 min, 30 min, 30 min, and 30 min | 7 times, 5 times, 5 times, and 5 times |
| LDTXS2102005 | 3 | 30 min, 20 min, and 20 min | 9 times, 7 times, and 5 times |
| Accompanying decoction | 2 | 30 min and 20 min | 7 times and 5 times |

**Table 7 Experimental results for the Liang Di Tang granule LDTXS2102004**

| Batch No. | Dry extract yield/% | Harpagoside | | Paeoniflorin | |
|---|---|---|---|---|---|
| | | Content/wt% | Transfer rate/% | Content/wt% | Transfer rate/% |
| LDTXS2102004 (three extractions) | 49.66 | 0.0697 | 69.00 | 1.4434 | 64.73 |
| LDTXS2102004 (the fourth extraction) | 4.99 | 0.005 | 4.95 | 0.112 | 5.02 |

**Table 8 Investigation results of the number of extractions and the extraction time**

| Batch No. | Dry extract yield/% | Harpagoside | | Paeoniflorin | |
|---|---|---|---|---|---|
| | | Content/wt% | Transfer rate/% | Content/wt% | Transfer rate/% |
| LDTXS2102003 | 45.57 | 0.0708 | 70.10 | 1.4068 | 63.09 |
| LDTXS2102004 (three extractions) | 49.66 | 0.0697 | 69.00 | 1.4434 | 64.73 |
| LDTXS2102005 | 47.68 | 0.0749 | 74.16 | 1.3152 | 58.98 |
| RSD% | 4.29 | 3.82 | 3.82 | 4.76 | 4.76 |
| Accompanying decoction | 39.95 | 0.0700 | 69.31 | 1.2120 | 54.35 |

Results in Tables 7 and 8 show: (1) With the increase of the number of extractions, the extension of the extraction time (results of the first three extractions are calculated), and the increase of the water amount, the dry extract yield and the contents of harpagoside and paeoniflorin as major quality indexes increase compared with the accompanying decoction, but there is little difference among different batches (RSD: less than 5%). (2) According to the results of the fourth extraction, the dry extract yield and the contents of harpagoside and paeoniflorin as major quality indexes all are not zero, but there is a low dry extract yield of about 5%. (3) Based on the analysis of the verification test of increasing the number of extractions, the results of the accompanying decoction, and the standards of the reference sample, it is believed that the major quality indexes (a dry extract yield of 45.57%, a harpagoside transfer rate of 70.10%, and a paeoniflorin transfer rate of 63.09%) realize the target of extraction for the Liang Di Tang granule, fall within the standard ranges of the reference sample, and are higher than the corresponding quality indexes of the accompanying decoction.

### 1.4 Paeoniflorin and harpagoside dissolution tests

A traditional Chinese medicine extraction process involves the dissolution of various components in a solvent. To further validate the rationality of the extraction process for the Liang Di Tang granule, the changes in contents of paeoniflorin and harpagoside as representative components with the extraction time were monitored. Extraction time-integral area curves were plotted to determine the optimal extraction time.

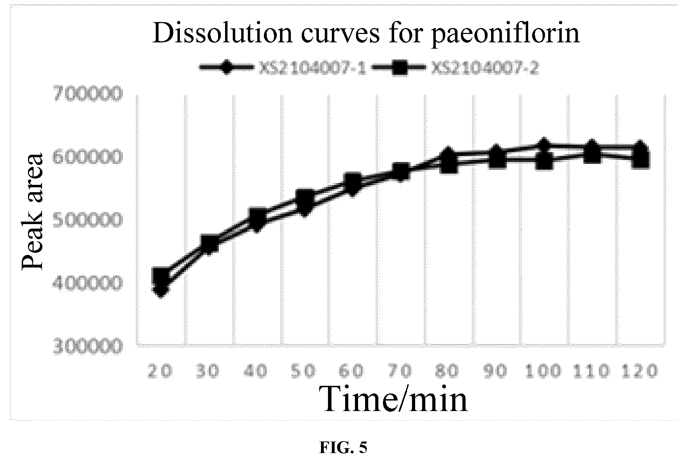

Test method: Two groups of the Chinese herbal slices for Liang Di Tang were weighed in a mass 1.5 times the above specified mass in each group and placed in round-bottomed flasks, respectively. Water was added at an amount 7 times the amount of the Chinese herbal slices for Liang Di Tang, and a resulting mixture was heated with an electric heating mantle. Timing was started once boiling, and slight boiling was maintained for 120 min. During the slight boiling, 5 mL of a medicinal liquid was collected by a pipette once every 10 min, filtered through a microporous filter membrane, and tested according to the method for determining the contents of paeoniflorin and harpagoside in the Liang Di Tang reference sample. According to test results, dissolution curves (extraction time-integral area) of the two components over the extraction time were plotted. The changes in contents of paeoniflorin and harpagoside in the medicinal liquid at different extraction times were compared to determine the change trends. A dissolution curve of harpagoside is shown in FIG. 4, and a dissolution curve of paeoniflorin is shown in FIG. 5.

As shown in FIG. 4 and FIG. 5, when the Chinese herbal slices for Liang Di Tang are subjected to extraction for 30 min, masses of harpagoside and paeoniflorin extracted are 90% and 78% of total masses of harpagoside and paeoniflorin that could be extracted, which further validates the optimized extraction process (3.3.4.4.1) determined based on the investigation on the extraction time for the Liang Di Tang granule (Batch No. LDTXS2102003). Compared with the optimal extraction time (a single extraction, 7 times of water, and 70 min), the 30 min extraction involves a high extraction rate and reasonable extraction parameters.

### 2. Research on a concentration process

Vacuum evaporation is a common concentration method for the modern traditional Chinese medicine preparations, and has advantages such as low temperature and high efficiency. Thus, vacuum evaporation was selected for the concentration to obtain the Liang Di Tang granule. Through a rotary evaporation experiment, an appropriate temperature range was determined for the vacuum concentration to prepare the Liang Di Tang preparation.

Test method: five traditional Chinese medicines (2.6 kg of wine-processed *Rehmanniae Radix,* 2.6 kg of *Scrophulariae Radix,* 1.3 kg of wine-processed *Paeoniae Radix Alba,* 1.3 kg of *Ophiopogon japonicus,* and 0.78 g of *Lycii Cortex*) were weighed and added to a 100 L extraction tank. Water was added thereto, and decoction was conducted 3 times. For the first decoction, water was added at an amount 7 times a total amount of the five traditional Chinese medicines, soaking was conducted for 40 min, and heat extraction was conducted for 30 min. For the second decoction, water was added at an amount 5 times the total amount of the five traditional Chinese medicines, and a resulting mixture was heated for 20 min. For the third decoction, water was added at an amount 5 times the total amount of the five traditional Chinese medicines, and a resulting mixture was heated for 20 min. Resulting extraction solutions were combined to obtain a combined extraction solution for later use.

About 4 kg of the combined extraction solution was taken and divided into 4 groups. The 4 groups each were weighed, placed in rotary evaporators, and concentrated at 60 °C, 70 °C, 80 °C, 90 °C, and 95 °C respectively to obtain thick concentrates (each with a weight of about 50 g). A concentration time and a vacuum degree were recorded, and contents of harpagoside and paeoniflorin in the thick concentrates were detected. A temperature of 70 °C to 90 °C was preferred for the vacuum concentration for the Liang Di Tang granule. Test results are shown in Table 9.

**Table 9 Investigation results for the vacuum concentration temperature**

| No. | Temperature ( °C) | Concentration time (min) | Harpagoside/% | Paeoniflorin/% |
|---|---|---|---|---|
| 1 | 60 | 108 | 0.060 | 1.226 |
| 2 | 70 | 81 | 0.060 | 1.243 |
| 3 | 80 | 81 | 0.059 | 1.198 |
| 4 | 90 | 64 | 0.058 | 1.188 |
| RSD% | - | - | 1.62 | 2.08 |

It can be seen from Table 9 that, when an extraction solution for the Liang Di Tang granule is vacuum-concentrated at a temperature of 60 °C to 90 °C, the quality indexes of harpagoside and paeoniflorin change slightly (RSD < 3.0%). However, with the increase of the concentration temperature, the concentration efficiency is improved. A temperature range of 70 °C to 90 °C is preferred for the vacuum concentration for the Liang Di Tang granule. When the vacuum concentration is conducted at 95 °C, it is prone to bumping and suckback due to the high temperature.

### 3. Research on a drying process

The vacuum belt drying has advantages such as low drying temperature, short drying time, small loss, and high efficiency, and is especially suitable for heat-sensitive materials with high viscosity and easy agglomeration. The Liang Di Tang granule formula contains wine-processed *Rehmanniae Radix, Ophiopogon japonicus,* and *Scrophulariae Radix* with many polysaccharide components, resulting in a material solution with a large viscosity. Therefore, the vacuum belt drying is selected for the Liang Di Tang granule.

There are many factors affecting the vacuum belt drying, mainly including a density of a medicinal liquid, a feeding rate, a drying time (namely, a speed of a track), and temperatures of heating zones. Therefore, these key parameters were investigated to determine a drying process for the Liang Di Tang granule.

### 3.1 Investigation of a density of a medicinal liquid

Mixed concentrates with different concentrations (density: 1.26 g/mL to 1.35 g/mL) for the Liang Di Tang granule were taken and dried in a vacuum belt dryer to obtain powders. A test process was observed. Powder discharged was weighed, and a powder yield was calculated.

According to results, when a medicinal liquid to be dried for the Liang Di Tang granule has a density of 1.26 g/mL to 1.35 g/mL, the loading is smooth, the spray-coating is uniform, there is slight adhesion, and a resulting dry powder has a prominent state. When a medicinal liquid to be dried for the Liang Di Tang granule has a density of 1.26 g/mL, a coating of the medicinal liquid is thin, and a drying efficiency is slightly low. When a medicinal liquid to be dried for the Liang Di Tang granule has a density of 1.36 g/mL, the medicinal liquid is relatively viscous and could be loaded, but there is violent bubble appearing during the drying, resulting in a loss. Therefore, an appropriate density of a medicinal liquid to be dried for the Liang Di Tang granule range from 1.26 g/mL to 1.35 g/mL.

### 3.2 Investigation of a drying temperature

According to characteristics of a vacuum belt dryer and the following drying temperatures of vacuum belt drying for traditional Chinese medicine extract pastes: 95±5 °C in a first zone, 96±5 °C at a second zone, and 96±5 °C at a third zone, a drying test was carried out on a Liang Di Tang (batch No.) concentrate. Results showed that the major quality indexes of harpagoside and paeoniflorin in the Liang Di Tang granule are relatively stable at the above drying temperatures. Therefore, belt drying temperatures for the Liang Di Tang granule were set as follows: 95±5 °C in a first zone, 96±5 °C at a second zone, and 96±5 °C at a third zone.

### Example 4

### Experimental research on the stability of paeoniflorin

### 1. Experimental object

In view of the problem that a content of paeoniflorin was reduced in the existing preparation process for Liang Di Tang preparations, the relevant analysis was conducted. Analysis results showed that the loss of paeoniflorin was related to the decomposition of paeoniflorin caused by long-time vacuum concentration and the addition of *Asini Corii Colla* and dextrin affecting the dissolution of paeoniflorin. The influence of a heating time and a paeoniflorin concentration on a paeoniflorin content was investigated through a single-factor experiment. The influence of the addition of *Asini Corii Colla* and dextrin on the dissolution of paeoniflorin was also investigated through a contrast experiment.

### 2. Experimental method

### 2.1 Investigation of the thermal stability of paeoniflorin

### 2.1.1 Preparation of sample solutions

A specified amount of a paeoniflorin reference substance was accurately weighed and dissolved with methanol to obtain a paeoniflorin solution with a concentration of 1.8905 mg/mL, which was a stock solution. An appropriate amount of the stock solution was accurately taken and diluted with an appropriate amount of purified water to prepare the paeoniflorin solutions with different concentrations shown in Table 10 (which were numbered 1 to 5).

The paeoniflorin solutions 1 to 5 were placed in a water bath and heated at 85±5 °C. At 0.5 h, 1.0 h, 1.5 h, 2.0 h, 2.5 h, 3.0 h, 3.5 h, and 4.0 h of heating, samples were collected, i.e., sample solutions.

**Table 10 Preparation methods for paeoniflorin solutions of a series of concentrations**

| No. | 1 | 2 | 3 | 4 | 5 |
|---|---|---|---|---|---|
| Amount of the stock solution (mL) | 0.28 | 0.8 | 1.4 | 2.2 | 2.8 |
| Final volume (mL) | 10 | 5 | 5 | 5 | 5 |
| Paeoniflorin concentration (mg/mL) | 0.05 | 0.3 | 0.5 | 0.8 | 1.0 |

### 2.1.2 Preparation of test sample solutions

An appropriate amount of each sample solution prepared in section 2.1.1 was taken and purified water was added thereto to prepare test sample solutions 1 to 5. The test sample solutions were injected for testing. Preparation methods for the test sample solutions 1 to 5 were as follows:
Series 1: At different heating times (heating for 0.5 h, 1.0 h, 1.5 h, 2.0 h, 2.5 h, 3.0 h, 3.5 h, and 4.0 h), a paeoniflorin solution with a concentration of 0.05 mg/mL was pipetted and filtered through a 0.45-µm filter membrane.
Series 2: At different heating times (heating for 0.5 h, 1.0 h, 1.5 h, 2.0 h, 2.5 h, 3.0 h, 3.5 h, and 4.0 h), 0.3 mL of a paeoniflorin solution with a concentration of 0.3 mg/mL was accurately pipetted and mixed with accurate 0.7 mL of purified water. A resulting mixture was thoroughly shaken and filtered through a 0.45-µm filter membrane.
Series 3: At different heating times (heating for 0.5 h, 1.0 h, 1.5 h, 2.0 h, 2.5 h, 3.0 h, 3.5 h, and 4.0 h), 0.2 mL of a paeoniflorin solution with a concentration of 0.5 mg/mL was accurately pipetted and mixed with accurate 0.8 mL of purified water. A resulting mixture was thoroughly shaken and filtered through a 0.45-µm filter membrane.
Series 4: 0.1 mL of a paeoniflorin solution with a concentration of 0.8 mg/mL was accurately pipetted and mixed with accurate 0.9 mL of purified water. A resulting mixture was thoroughly shaken and filtered through a 0.45-µm filter membrane.
Series 5: At different heating times (heating for 0.5 h, 1.0 h, 1.5 h, 2.0 h, 2.5 h, 3.0 h, 3.5 h, and 4.0 h), 0.1 mL of a paeoniflorin solution with a concentration of 1.0 mg/mL was accurately pipetted and added to a 2 mL volumetric flask, and purified water was added thereto to the marked volume. A resulting mixture was thoroughly shaken and filtered through a 0.45-µm filter membrane.

### 2.1.3 Detection method

Chromatography conditions and system suitability testing: A chromatographic column with octadecylsilane-bonded silica gel as a filler was adopted. An acetonitrile-0.1% phosphoric acid solution (at a volume ratio of 14 : 86) was adopted as a mobile phase. A detection wavelength was 230 nm. A column temperature was 27 °C. A mobile phase flow rate was 1.0 mL/min. The number of theoretical plates was calculated to be 28,000 based on a peak of paeoniflorin.

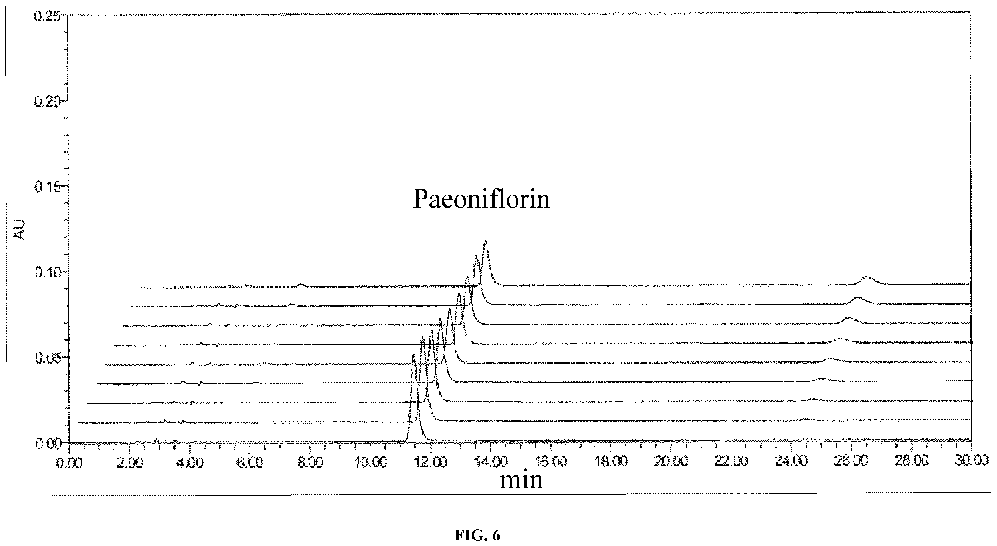

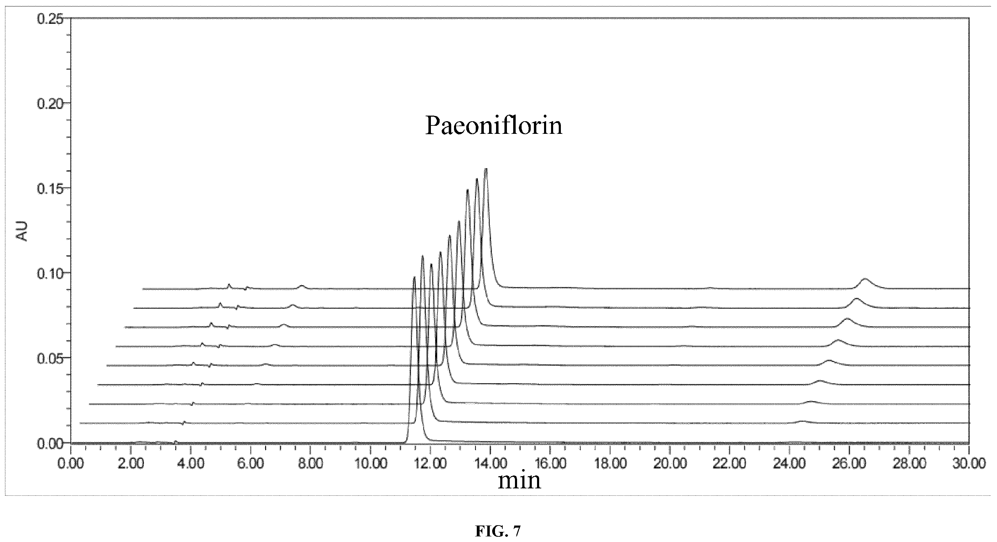

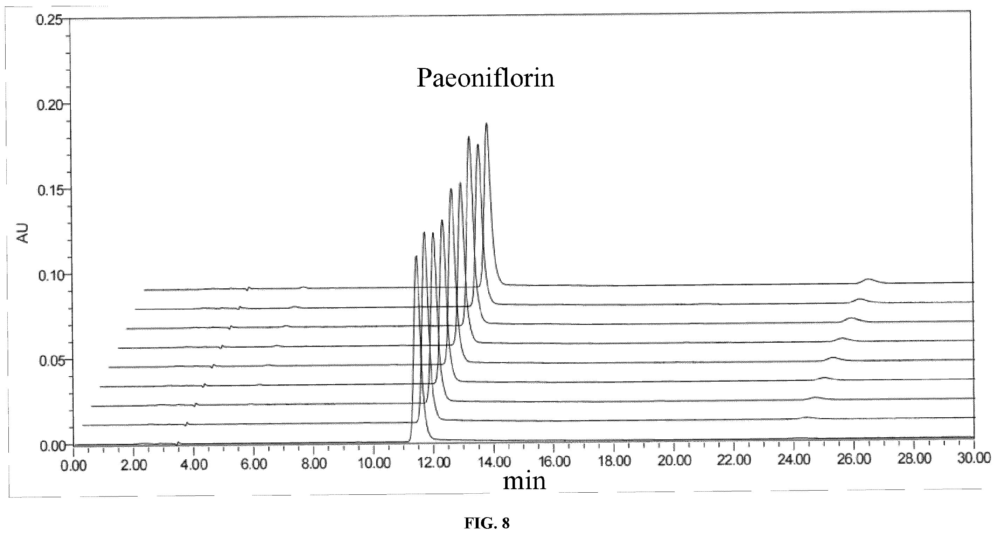

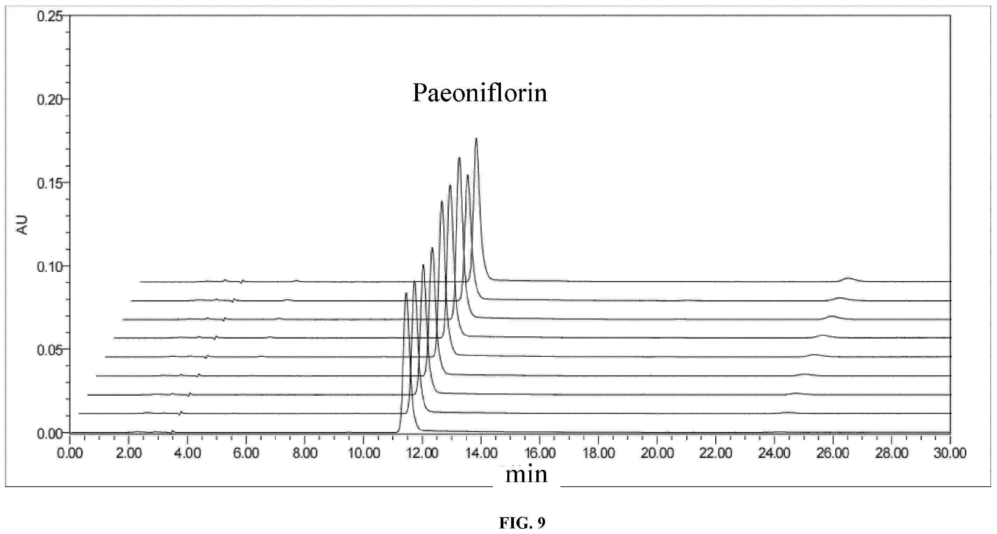

Determination: 10 µL of each of the test sample solutions of series 1 to series 5 prepared in section 2.1.2 was accurately pipetted and injected into a high-performance liquid chromatograph for testing to obtain liquid chromatography chromatograms of paeoniflorin in the test sample solutions of series 1 to series 5. Liquid chromatography chromatograms of paeoniflorin in the test sample solutions of series 1 are shown in FIG. 6. Liquid chromatography chromatograms of paeoniflorin in the test sample solutions of series 2 are shown in FIG. 7. Liquid chromatography chromatograms of paeoniflorin in the test sample solutions of series 3 are shown in FIG. 8. Liquid chromatography chromatograms of paeoniflorin in the test sample solutions of series 4 are shown in FIG. 9. Liquid chromatography chromatograms of paeoniflorin in the test sample solutions of series 5 are shown in FIG. 10.

### 2.1.4 Data analysis

With a heating time as an x-coordinate and a paeoniflorin area % as a y-coordinate, change curves of paeoniflorin solutions of different concentrations with a heating time were plotted. The influence of a concentration and a heating time on the stability of paeoniflorin was analyzed.

2.2 Investigation of influence of dextrin and *Asini Corii Colla* on the dissolution of paeoniflorin

### 2.2.1 Experimental method

Preparation of sample solutions: 0.53 mL of the paeoniflorin stock solution (1.890 mg/mL) was accurately taken and added to a 50 mL volumetric flask, water was added thereto to the marked volume, and the volumetric flask was thoroughly shaken to obtain a paeoniflorin solution with a concentration of 0.02 mg/mL. Appropriate amounts of the paeoniflorin solution were taken and added to test tubes each with a stopper, and dextrin and *Asini Corii Colla* were separately added to allow different treatments. The different treatments are shown in Table 11. After thorough mixing, pH values of solutions in test tubes were determined. Heating was conducted at 85 °C for 48 h with each test tube stoppered to obtain the sample solutions. At 4 h, 22 h, 32 h, and 48 h of heating, samples were collected and tested for a paeoniflorin content.

**Table 11 Preparation methods of samples for investigating the influence of dextrin and Asini Corii Colla on the dissolution of paeoniflorin**

| No. | Name | Treatment |
|---|---|---|
| 1 | *Asini Corii Colla* + heating | 0.25 g of *Asini Corii Colla* was weighed and added to the paeoniflorin solution, and a resulting mixture was heated in a water bath at 90 °C for 20 min. |
| 2 | *Asini Corii Colla* | 0.25 g of *Asini Corii Colla* was weighed and added to the paeoniflorin solution, and a resulting mixture was subjected to an ultrasonic treatment for 20 min. |
| 3 | *Asini Corii Colla* + dextrin | 0.4 g of *Asini Corii Colla* and 0.25 g of dextrin were weighed and added to the paeoniflorin solution, and a resulting mixture was heated in a water bath for 20 min. |
| 4 | Dextrin | 0.25 g of dextrin was weighed and added to the paeoniflorin solution, and a resulting mixture was heated in a water bath for 20 min. |

Preparation of test sample solutions: 2 mL of a sample solution was accurately pipetted and placed in an Erlenmeyer flask with a stopper, and 8 mL of acetonitrile was accurately added thereto. The Erlenmeyer flask was thoroughly shaken, weighed, and stoppered. A resulting mixture was subjected to an ultrasonic treatment (800 W, 40 kHz) for 20 min, and then naturally cooled. The Erlenmeyer flask was weighed once again, and an 80% (v/v) aqueous acetonitrile solution was supplemented to make up for a weight loss. The Erlenmeyer flask was thoroughly shaken, and a resulting mixture was then filtered with a 0.45-µm filter membrane to obtain a filtrate, which was a test sample solution.

### 2.2.3 Detection method: Detection was carried out in accordance with section 2.1.3.

### 2.2.4 Data analysis

A concentration of paeoniflorin in a sample was calculated according to equation (1). Concentrations of paeoniflorin in different samples were compared, and RSD% was calculated. Concentration of paeoniflorin in a sample (C) = measured amount (µg)/volume of a test sample loaded (µL) × final volume of a test sample solution (mL)/volume of the test sample solution (mL)

### 3. Experimental results

### 3.1 Investigation of the thermal stability of paeoniflorin

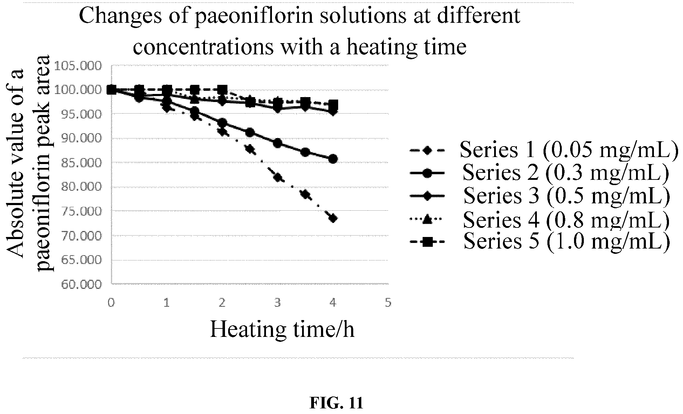

Samples were collected from the series of paeoniflorin solutions with different concentrations at different heating times and tested to obtain a peak area of each sample. An initial (0 h) peak area of each series was set as 100. The peak area of each sample was divided by the initial peak area of the respective series to obtain a relative peak integral area of each sample. Test results are shown in Table 12. With a heating time as an x-coordinate and a relative peak integral area of paeoniflorin in a paeoniflorin solution at each concentration as a y-coordinate, a time-relative peak integral area change curve was plotted. Test results of changes of paeoniflorin solutions at different concentrations with a heating time are shown in FIG. 11.

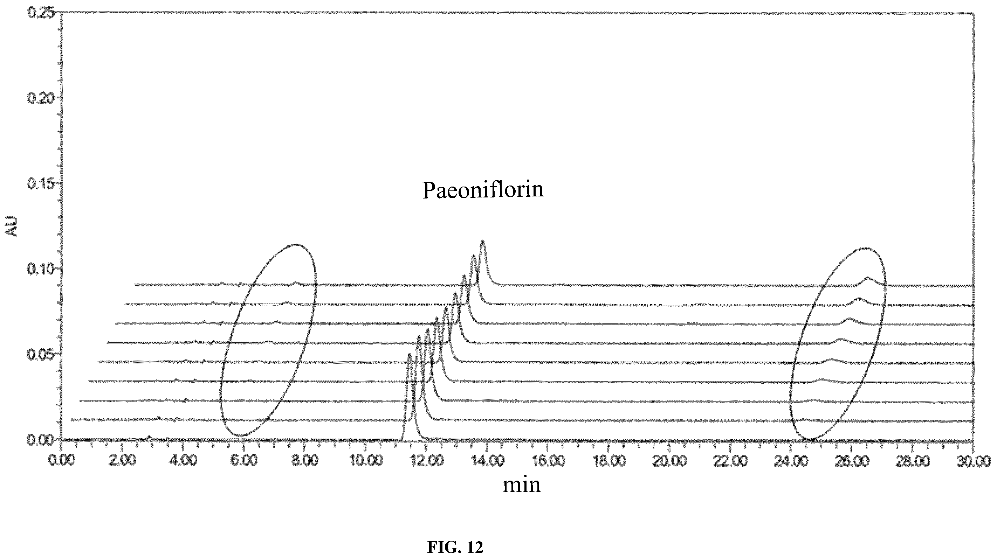

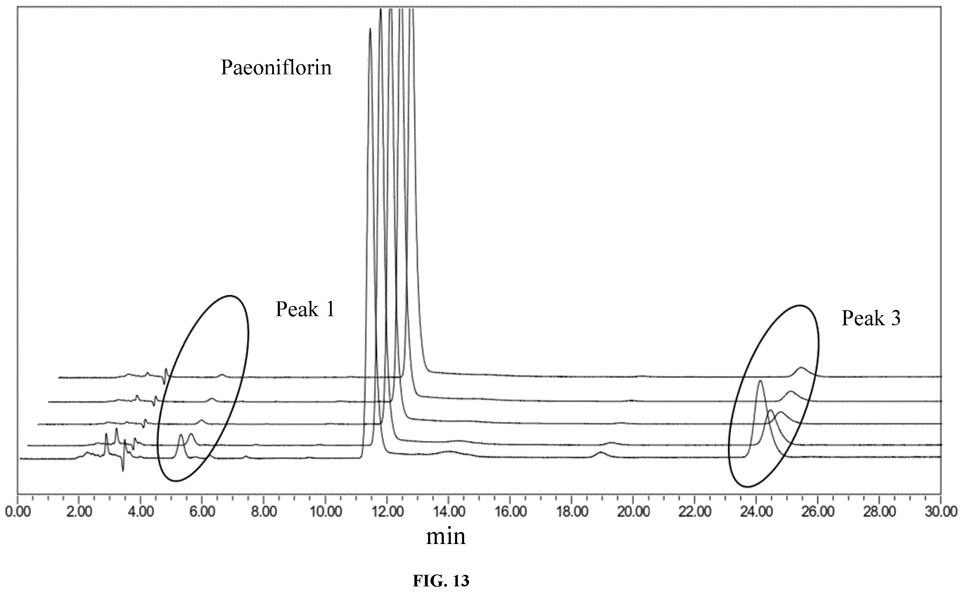

In order to further study the relationships of the stability of a paeoniflorin solution with a concentration and a heating time, chromatographic changes of a paeoniflorin solution with a heating time and chromatographic changes of paeoniflorin solutions at different concentrations under a same heating time were analyzed. Results are shown in FIG. 12 and FIG. 13. FIG. 12 shows chromatographic changes of a paeoniflorin solution at a concentration of 0.05 mg/mL when heated for 4 h, where the chromatograms from bottom to top correspond to heating times of: 0 h, 0.5 h, 1 h, 1.5 h, 2 h, 2.5 h, 3 h, 3.5 h, and 4 h, respectively. FIG. 13 shows chromatographic changes of paeoniflorin solutions at different concentrations when heated for 4 h, where the chromatograms from bottom to top correspond to concentrations of: 0.05 mg/mL, 0.3 mg/mL, 0.5 mg/mL, 0.8 mg/mL, and 1.0 mg/mL, respectively.

**Table 12 Test results of changes of paeoniflorin solutions at different concentrations (peak area) with heating times**

| | Paeoniflorin concentration series | Series 1 (0.05 mg/mL) | Series 2 (0.3 mg/mL) | Series 3 (0.5 mg/mL) | Series 4 (0.8 mg/mL) | Series 5 (1.0 mg/mL) |
|---|---|---|---|---|---|---|
| Heating time/h | 0 | 100.000 | 100.000 | 100.000 | 100.000 | 100.000 |
| | 0.5 | 100.000 | 98.262 | 98.628 | 100.000 | 100.000 |
| | 1 | 96.170 | 97.537 | 98.881 | 100.000 | 100.000 |
| | 1.5 | 94.446 | 95.524 | 97.956 | 98.060 | 100.000 |
| | 2 | 91.295 | 93.091 | 97.499 | 98.341 | 100.000 |
| | 2.5 | 87.717 | 91.121 | 97.179 | 97.845 | 97.494 |
| | 3 | 81.853 | 88.913 | 96.021 | 97.530 | 97.239 |
| | 3.5 | 78.341 | 87.053 | 96.386 | 97.410 | 97.428 |
| | 4 | 73.433 | 85.660 | 95.387 | 96.690 | 96.887 |
| | pH | 6.67 | 6.49 | 6.54 | 6.39 | 6.30 |

It can be seen from Table 12 and FIG. 11 to FIG. 13 that: the stability of paeoniflorin during heating is related to a concentration of a paeoniflorin solution. After the paeoniflorin solutions with concentrations of 0.05 mg/mL, 0.3 mg/mL, 0.5 mg/mL, 0.8 mg/mL, and 1.0 mg/mL are heated for 4 h, the corresponding peak areas of paeoniflorin decrease to 73.43%, 85.66%, 95.39%, 96.69%, and 96.89% of the initial values, respectively. Two significant impurity peaks are added in chromatograms of paeoniflorin solutions with low concentrations (lower than 0.5 mg/mL). An area of an impurity peak increases with the extension of a heating time and decreases with the increase of a concentration of a paeoniflorin solution. It indicates that the decomposition of paeoniflorin during heating is related to the heating time and the concentration of paeoniflorin. A longer heating time results in a lower concentration of paeoniflorin, indicating a more serious decomposition of paeoniflorin.

3.2 Investigation of influence of dextrin and *Asini Corii Colla* on the dissolution of paeoniflorin: A concentration of paeoniflorin in each sample was calculated. Concentrations of paeoniflorin in different samples were investigated in parallel, and RSD% was calculated. Results are shown in Table 13.

**Table 13 Investigation of influence of dextrin and Asini Corii Colla on the dissolution of paeoniflorin (n = 2)**

| Name | Measured amount (µg) | Average (µg) | Concentration (mg/mL) |
|---|---|---|---|
| Original paeoniflorin solution | 0.315 | 0.316 | 0.158 |
| | 0.317 | | |
| Paeoniflorin + *Asini Corii Colla* + heating | 0.306 | 0.3135 | 0.15675 |
| | 0.321 | | |
| Paeoniflorin + *Asini Corii Colla* + ultrasound treatment | 0.298 | 0.303 | 0.1515 |
| | 0.308 | | |
| Paeoniflorin + *Asini Corii Colla* + dextrin | 0.282 | 0.284 | 0.142 |
| | 0.286 | | |
| Paeoniflorin + dextrin | 0.313 | 0.3095 | 0.15475 |
| | 0.306 | | |
| RSD% | | | 3.76 |

It can be seen from Table 13 that a concentration of paeoniflorin in a sample added with both *Asini Corii Colla* and dextrin is not significantly different from a concentration of paeoniflorin in the original paeoniflorin solution (RSD% < 5.0).

### 4. Experimental conclusion

1. The decomposition of paeoniflorin in a paeoniflorin solution during heating is related to the heating time and the concentration of paeoniflorin. A longer heating time results in a lower paeoniflorin concentration, indicating a more serious decomposition of paeoniflorin. The further analysis indicates that a concentration of paeoniflorin is related to a pH value. Paeoniflorin contains a large number of hydroxyl groups. A higher paeoniflorin concentration results in a lower pH value. Some studies have shown that paeoniflorin is relatively stable in an acidic environment with a pH value of 3 to 5. When a pH value is higher than 6, the stability of paeoniflorin decreases significantly. A Liang Di Tang extract liquid has a pH value of about 6, and paeoniflorin is unstable during a concentration process thereof. Therefore, the vacuum concentration would cause the decomposition of paeoniflorin.
2. The addition of *Asini Corii Colla* and dextrin does not have a significant impact on the dissolution of paeoniflorin (RSD% < 5), indicating that the encapsulation of *Asini Corii Colla* and dextrin does not affect the dissolution of paeoniflorin.
3. The decomposition of paeoniflorin is mainly related to a pH value of a medicinal liquid. Thus, a pH value of a medicinal liquid needs to be controlled during the extraction and concentration processes for Liang Di Tang. The specific method for controlling the pH value needs to be further studied.

### Example 5

### Preparation and quality standard for a Liang Di Tang granule

### 1. Preparation of the Liang Di Tang granule

Soaking: 208.0 kg of wine-processed *Rehmanniae Radix,* 208.0 kg of *Scrophulariae Radix,* 104.0 kg of wine-processed *Paeoniae Radix Alba,* 104.0 kg of *Ophiopogon japonicus,* and 62.4 kg of *Lycii Cortex* were weighed and added to an extraction tank, water was added at an amount 7 times an amount of the Chinese herbal slices, and soaking was conducted for 40 min.

Extraction: after being heated to 100 °C, a resulting mixture was subjected to a first extraction for 30 min, and a resulting medicinal liquid was discharged. Water was added at an amount 5 times the amount of the Chinese herbal slices to the residue, and after being heated to 100 °C, a resulting mixture was subjected to a second extraction for 20 min, and a resulting medicinal liquid was discharged. Water was added at an amount 5 times the amount of the Chinese herbal slices to the residue, and after being heated to 100 °C, a resulting mixture was subjected to a third extraction for 20 min, and a resulting medicinal liquid was discharged.

Centrifugation: the medicinal liquids were centrifuged separately by a centrifuge (separation speed: 60 L/min, and residue discharge: 10 min/time).

Fine filtration: each centrifugate was filtered through a plate and frame filter (a filter membrane had a pore size of 5 µm to 15 µm).

*Asini Corii Colla* liquefaction: 62.4 kg of *Asini Corii Colla* was weighed and placed in a jacketed pot, and water was added in a mass 4 times a mass of the *Asini Corii Colla.* A resulting mixture was heated until boiling with continuous stirring to obtain a homogeneous *Asini Corii Colla* liquid. The foams and impurities in an upper layer were removed, and a resulting liquid was filtered through a 100-mesh sieve to obtain an *Asini Corii Colla* liquid for later use.

Dextrin dissolution: 40 kg of dextrin was weighed and poured into a stainless-steel barrel, water was added in a mass 4 times a mass of the dextrin, and a resulting mixture was stirred until complete dissolution to obtain an aqueous dextrin solution for later use.

Separate concentration of extraction solutions: Under vacuum, each medicinal liquid produced after filtration was fed into a vacuum concentrator. A steam valve was opened, and heating was started. Vacuum concentration was conducted at a temperature of 85±5 °C, a vacuum degree of 0.045±0.015 MPa, and a steam pressure of 0.04±0.01 MPa to obtain a concentrate with a density of greater than or equal to 1.10 g/mL and less than 1.20 g/mL. During the concentration, a boiling state and a temperature were observed at any time, and the vacuum degree and the steam pressure were then adjusted. The concentrate was introduced into a storage tank/transfer barrel and weighed for later use.

Mixing and concentration: Under vacuum, concentrates resulting from the three extractions, the *Asini Corii Colla* liquid, and the aqueous dextrin solution were introduced into a single-effect concentrator, and a resulting mixture was further concentrated under conditions for the above separate concentration to obtain a thick paste with a density of 1.25 g/mL to 1.36 g/mL (20 °C). The thick paste was transferred into a transfer barrel and weighed for later use.

Drying: The thick paste was dried in a vacuum belt dryer to obtain a powder. Parameters of the vacuum belt dryer were as follows: a feeding temperature: 80 °C; a feeding rate: 18 L/h; a running speed of a track: 16±5 cm/min; an angle of a swingarm: 70°; a speed of a material-distributing motor: 40 r/min; heating temperatures in a first zone, a second zone, a third zone, and a fourth zone being 95±5 °C, 96±5 °C, 96±5 °C, and 30±5 °C (cooling), respectively; a speed of a cutter: 10 s/time; and a vacuum degree: 0.097 MPa to 0.1 MPa. The powder was collected in a transfer barrel and packaged for later use.

Granulation: The powder produced after the belt drying was taken, weighed, transferred to a granulator, and granulated to obtain a granule for later use. Parameters for the granulator were as follows: a pressure of a press roller: 16 MPa; rotational speeds of a screw feeder: a vertical rotational speed being 24 r/min, and a horizontal rotational speed being 120 r/min; a rotational speed of the press roller: 6 r/min; and a granule sizing speed: 130 r/min. During the granulation, the conditions of the granulator and the appearances of particles were observed, and the granulation parameters were adjusted appropriately. The powder was fed and the particles were collected in time. The adjustment for the granulation parameters was recorded.

A power supply for a vibrating sieve was turned on. Particles produced after the granulation were placed in a hopper, and a valve of the hopper was adjusted, such a feeding rate was adopted that the qualified particles were completely separated from a fine powder. The particles were placed in a transfer barrel for later use. The fine powder was re-granulated. 400 kg of the Liang Di Tang granule was prepared in total.

Packing: The packaging was conducted with a pharmaceutical composite film according to the specification of 12 g/bag, and a total of 330,000 bags were prepared.

### 2. Quality standard for the Liang Di Tang granule

2.1 Formula: 520 g of wine-processed *Rehmanniae Radix,* 520 g of *Scrophulariae Radix,* 260 g of wine-processed *Paeoniae Radix Alba,* 260 g of *Ophiopogon japonicus,* 156 g of *Lycii Cortex,* and 156 g of *Asini Corii Colla.*

Appearance: This product was a light-gray to dark-gray granule with a sweet and slightly-bitter taste.

### 2.2 Characteristic chromatograms

Preparation of a reference substance solution: Appropriate amounts of a paeoniflorin reference substance and a harpagoside reference substance were weighed accurately, dissolved in methanol, and diluted to a specified volume to obtain the reference substance solution. In the reference substance solution, a concentration of the paeoniflorin reference substance was 200 µg/mL, and a concentration of the harpagoside reference substance was 30 µg/mL.

Preparation of a test sample solution: The Liang Di Tang granule prepared in step (1) was finely ground to obtain a powder. About 1 g of the powder was weighed accurately and placed in an Erlenmeyer flask with a stopper. 1 g of diatomaceous earth was added thereto, a resulting mixture was mixed to be uniform, and 25 mL of an 80 v/v% aqueous acetonitrile solution was accurately added thereto. The Erlenmeyer flask was weighed. A resulting mixture was subjected to an ultrasonic treatment (with a power of 800 W and a frequency of 40 kHz) for 20 min, and then cooled. The Erlenmeyer flask was weighed once again, and an 80 v/v% aqueous acetonitrile solution was supplemented to make up for a weight loss. The Erlenmeyer flask was shaken thoroughly, and a resulting mixture was then filtered. 15 mL of a resulting filtrate was accurately collected and subjected to evaporation to dryness to obtain a residue. The residue was dissolved with 10% methanol and transferred to a 10 mL volumetric flask, and 10% methanol (v/v) was added thereto to the marked volume. The volumetric flask was thoroughly shaken, and a resulting mixture was then filtered. A resulting filtrate was collected, which was the test sample solution.

Determination: 10 µL of each of the reference substance solution and the test sample solution was accurately pipetted and injected into a high-performance liquid chromatograph for testing. A chromatogram within 60 min was recorded to obtain a contrasted characteristic chromatogram (as shown in FIG. 2).

Chromatography conditions and system suitability testing: Octadecylsilane-bonded silica gel was adopted as a filler. Methanol was adopted as a mobile phase A and a 0.05 v/v% aqueous phosphoric acid solution was adopted as a mobile phase B. Gradient elution was conducted according to the gradient elution program shown in Table 1. A detection wavelength was 254 nm. A column temperature was 28 °C. The number of theoretical plates was calculated to be 30,100 based on a peak of paeoniflorin.

As shown in FIG. 2, 10 characteristic peaks are present in a test sample characteristic chromatogram, 2 peaks of which should each have a retention time consistent with a peak of a corresponding reference substance. A peak corresponding to the paeoniflorin reference substance is taken as an S peak. A relative retention time of each characteristic peak relative to the S peak is calculated, and the relative retention time should be within ±10% of a specified value. The specified value is as follows: 0.22 (peak 1), 0.26 (peak 2), 0.31 (peak 3), 0.33 (peak 4), 1.00 (peak 5), 1.47 (peak 6), 1.54 (peak 7), 1.82 (peak 8), 1.91 (peak 9), and 2.22 (peak 10). The peaks 1 to 3 are attributed to unknown peaks. The peak 4 is attributed to gallic acid. The peak 5 is attributed to paeoniflorin. The peak 7 is attributed to verbascoside. The peak 8 is attributed to angoroside C. The peak 9 is attributed to cinnamic acid. The peak 10 is attributed to harpagoside.

### 2.3 Water content and dry extract yield

Identification: (1) 4 g of the sample powder was taken and 30 mL of methanol was added thereto. A resulting mixture was subjected to an ultrasonic treatment for 30 min (power: 400 W to 600 W), and then filtered to obtain a filtrate. The filtrate was concentrated to 5 mL to obtain a test sample solution. 1.5 g of *Lycii Cortex* as a control medicine was taken to prepare a control medicine solution according to the preparation method for the test sample solution. 10 µL of each of the above two solutions was pipetted and spotted on a same silica gel G thin-layer chromatography plate. With a mixed solvent of cyclohexane, ethyl acetate, and formic acid at a volume ratio of 5 : 5 : 0.4 as a developing solvent, developing was conducted. The silica gel G thin-layer chromatography plate was taken out, air-dried, and inspected under an ultraviolet lamp (365 nm). Fluorescent spots in a same color appeared at a position in the chromatography plate of the test sample corresponding to the chromatography plate of the control medicine.

Detection: A dry extract yield ranged from 40.0% to 55.0%.

### 2.4 Determination of contents of harpagoside and paeoniflorin

Chromatography conditions and system suitability testing: A chromatographic column with octadecylsilane-bonded silica gel as a filler was adopted. Methanol was adopted as a mobile phase A and a 0.05% aqueous phosphoric acid solution was adopted as a mobile phase B. A gradient elution program shown in Table 1 was adopted. A detection wavelength was 254 nm. A column temperature was 28 °C. The number of theoretical plates was calculated to be 2,910 based on a peak of paeoniflorin.

Preparation of a reference substance solution: A paeoniflorin reference substance and a harpagoside reference substance were weighed accurately, dissolved in methanol, and diluted to a specified volume to obtain the reference substance solution in which a concentration of harpagoside was 30 µg/mL and a concentration of paeoniflorin was 200 µg/mL.

The test sample solution was prepared according to steps in section 2.2.

Determination: 10 µL of each of the reference substance solution and the test sample solution was accurately pipetted, injected into a high-performance liquid chromatograph, and subjected to HPLC test to obtain the contents of harpagoside and paeoniflorin.

Each bag (12 g) of the Liang Di Tang granule contained 4.0 mg to 8.0 mg of *Scrophulariae Radix* in terms of harpagoside and 33.0 mg to 62.0 mg of wine-processed *Paeoniae Radix Alba* in terms of paeoniflorin.

### 2.5 Determination of a content of Asini Corii Colla (L-hydroxyproline and glycine)

Chromatography conditions and system suitability testing: A chromatographic column with octadecylsilane-bonded silica gel as a filler was adopted. An acetonitrile-0.1 mol/L sodium acetate solution (a pH was adjusted with acetic acid to 6.5) (a volume ratio of the acetonitrile to the sodium acetate solution was 7 : 93) was adopted as a mobile phase A, and an 80% (v/v) aqueous acetonitrile solution was adopted as a mobile phase B. Gradient elution was conducted according to the gradient elution program in Table 2. A flow rate was 0.8 mL/min. A detection wavelength was 254 nm. A column temperature was 43 °C. The number of theoretical plates was calculated to be 6,100 based on a peak of L-hydroxyproline.

Preparation of a reference substance solution: Appropriate amounts of an L-hydroxyproline reference substance and a glycine reference substance were weighed accurately and dissolved with a 0.1 mol/L hydrochloric acid solution to prepare a mixed solution containing 60 µg of L-hydroxyproline and 130 µg of glycine per 1 mL.

Preparation of a test sample solution: 1.5 g of the sample powder was weighed accurately and added to a 25 mL volumetric flask, 20 mL of a 0.1 mol/L hydrochloric acid solution was added thereto. A resulting mixture was subjected to an ultrasonic treatment for 30 min, and then cooled. A 0.1 mol/L hydrochloric acid solution was added thereto to the marked volume, and the volumetric flask was fully shaken to obtain a solution. 2 mL of the solution was accurately taken and added to a 10 mL ampoule, then 2 mL of hydrochloric acid was added thereto, and a resulting mixture was subjected to hydrolysis at 150 °C for 1 h. A resulting hydrolysis system was cooled, transferred to an evaporation dish, and washed with 10 mL of water in batches. Resulting washing solutions were combined in an evaporation dish, and subjected to evaporation to dryness to obtain a residue. The residue was dissolved with a 0.1 mol/L hydrochloric acid solution, and a resulting solution was transferred to a 25 mL volumetric flask, and diluted with a 0.1 mol/L hydrochloric acid solution to the marked volume, followed by thoroughly shaking.

Derivation: 1 mL of each of the reference substance solution and the test sample solution was accurately taken and added to 25 mL test tubes each with a stopper, and 0.5 mL of a solution of 0.1 mol/L PITC in acetonitrile and 0.5 mL of a solution of 1 mol/L triethylamine in acetonitrile were added to each test tube. The test tubes were thoroughly shaken and placed at room temperature for 1 h. 3 mL of a 50% (v/v) aqueous acetonitrile solution was added to each test tube, and the test tubes were thoroughly shaken. 5 mL of n-hexane was added to each test tube, and the test tubes were shaken and placed for 10 min. Resulting solutions in lower layers were collected and filtered through a microporous filter membrane to obtain filtrates, which were a derived reference substance solution and a derived test sample solution, respectively.

Determination: 5 µL of each of the derived reference substance solution and the derived test sample solution was accurately pipetted, injected into a high-performance liquid chromatograph, and subjected to HPLC test to obtain the contents of L-hydroxyproline and glycine.

Each bag (12 g) of the Liang Di Tang granule contained 0.10 g to 0.20 g of *Asini Corii Colla* in terms of L-hydroxyproline and 0.21 g to 0.39 g of *Asini Corii Colla* in terms of glycine.

2.6 Functions and indication: The Liang Di Tang granule exhibits the function of nourishing yin and clearing heat and the function of nourishing blood and normalizing menstrual cycle. The Liang Di Tang granule has the indication of Yin deficiency with blood-heat syndrome, which is characterized by the following symptoms: early menstruation, scanty and red menstrual flow, heat sensation in hands and feet, irritation and insomnia, red tongue with scanty coating, and thin and rapid pulse.

Usage and dosage: The Liang Di Tang granule is dissolved in boiling water and then taken orally. The Liang Di Tang granule is taken 3 times daily with two bags each time.

Specification: 12 g/bag.

Storage: The Liang Di Tang granule should be stored with sealed in a dry environment.

### Example 6

Three consecutive batches of commercial-scale production testing for the Liang Di Tang granule

### 1. Experimental process

### (1) Soaking and extraction

208.0 kg of wine-processed *Rehmanniae Radix,* 208.0 kg of *Scrophulariae Radix,* 104.0 kg of wine-processed *Paeoniae Radix Alba,* 104.0 kg of *Ophiopogon japonicus,* and 62.4 kg of *Lycii Cortex* were weighed and divided into two groups, and the two groups were placed in two extraction tanks, respectively. Water was added at an amount 7 times an amount of the Chinese herbal slices, and soaking was conducted for 40 min. Water was added at an amount 5 times the amount of the Chinese herbal slices, a resulting mixture was subjected to a first heat extraction at 100 °C for 20 min, and a resulting medicinal liquid was discharged. Water was then added at an amount 5 times the amount of the Chinese herbal slices to the residue, a resulting mixture was subjected to a second heat extraction at 100 °C for 20 min, and a resulting medicinal liquid was discharged. Water was then added at an amount 4 times to 5 times the amount of the Chinese herbal slices to the residue, a resulting mixture was subjected to a third heat extraction at 100 °C for 20 min, and a resulting medicinal liquid was discharged.

### (2) Centrifugation and fine filtration

The medicinal liquids were centrifuged by a centrifuge (separation speed: 60 L/min, and residue discharge: 10 min/time) separately. The number of residue discharges was recorded. Each centrifugate was filtered through a plate and frame filter (a filter membrane had a pore size of 5 µm to 15 µm, and when a pressure exceeded 0.2 MPa, a fine filter plate was replaced). The number of plate replacements was recorded.

### (3) Concentration

Under vacuum, each medicinal liquid produced after filtration was fed into a vacuum concentrator. A steam valve was opened, and heating was started. Vacuum concentration was conducted at a temperature of 85±5 °C, a vacuum degree of 0.055±0.015 MPa, and a steam pressure of lower than or equal to 0.09 MPa to obtain a concentrate with a density of greater than or equal to 1.10 g/mL (20 °C). During the concentration, a boiling state and a temperature were observed at any time, and the vacuum degree and the steam pressure were adjusted. The concentrate was introduced into a storage tank/transfer barrel and weighed for later use.

### (4) Liquefaction of Asini Corii Colla and dissolution of dextrin

62.4 kg of *Asini Corii Colla* was weighed and placed in a jacketed pot, and water was added in a mass 6 times a mass of the *Asini Corii Colla.* A reuslting mixture was heated until boiling, with continuous stirring, to obtain a homogeneous *Asini Corii Colla* liquid. The foams and impurities in an upper layer were removed, and a resulting liquid was filtered through a 120-mesh sieve to obtain an *Asini Corii Colla* liquid for later use.

40 kg of dextrin (10% of a dry extract) was weighed and poured into a stainless-steel barrel, water was added in a mass 4 times a mass of the dextrin, and a resulting mixture was stirred until complete dissolution to obtain a dextrin solution for later use.

### (5) Mixing and concentration

Three concentrates, the *Asini Corii Colla* liquid, and the dextrin solution were introduced into a single-effect concentrator, and a reuslting mixture was further concentrated under the concentration conditions described in step (3) to obtain a thick paste with a density of 1.25 g/mL to 1.36 g/mL (20 °C). The thick paste was transferred into a transfer barrel and weighed for later use.

### (6) Drying

The thick paste was introduced into a storage tank, heated to 90±5 °C, sterilized at 90±5 °C for 60 min, and cooled to 80±5 °C. A sterilized thick paste was dried in a vacuum belt dryer to obtain a powder. Parameters of the vacuum belt dryer were as follows: a feeding temperature: 83±2 °C; a feeding rate: 17.5±2.5 L/h; a running speed of a track: 16±5 cm/min; an angle of a swingarm: 80°; a speed of a material-distributing motor: 40 r/min; heating temperatures in a first zone, a second zone, a third zone, and a fourth zone being 95±5 °C, 96±5 °C, 96±5 °C, and 30±5 °C (cooling), respectively; a speed of a cutter: 20 s/time; and a vacuum degree: 0.097 MPa to 0.1 MPa. The powder was collected in a transfer barrel and packaged for later use.

### (7) Granulation

The powder produced after the belt drying was taken, weighed, transferred to a granulator, and granulated to obtain a granule. Parameters for the granulator were as follows: a pressure of a press roller: 17 MPa; rotational speeds of a screw feeder: a vertical rotational speed being 20 r/min, and a horizontal rotational speed being 100 r/min; a rotational speed of the press roller: 5.5 r/min; and a granule sizing speed: 130 r/min. During the granulation, the conditions of the granulator and the appearances of particles were observed, and the granulation parameters were adjusted appropriately. The powder was fed and the particles were collected in time. A power supply for a vibrating sieve was turned on. Particles produced after the granulation were placed in a hopper, and a valve of the hopper was adjusted, such a feeding rate was adopted that qualified particles were completely separated from a fine powder. The particles were placed in a transfer barrel for later use. The fine powder was re-granulated. The adjustment for the granulation parameters was recorded. A sample was collected and tested for a water content and contents of paeoniflorin and harpagoside.

### (8) Subpacking

The Liang Di Tang granule was packed with a composite film at a specification of 12 g/bag. The 12 g/bag indicated that an amount in each bag was controlled at 12.00 g to 12.50 g (A yield rate of the subpacking was calculated, and a sample was collected and tested for a granule load, a particle size, a water content, a paeoniflorin content, a harpagoside content, a characteristic chromatogram, etc.)

### 2. Experimental results

According to the three consecutive batches of commercial-scale production testing for the Liang Di Tang granule, the process route was reasonable, the major devices for extraction, centrifugation, fine filtration, concentration, drying, granulation, etc. exhibited excellent compatibility, the key process parameters were in line with the controlled ranges, and the entire testing process was smooth. Three batches of Liang Di Tang granules produced had yields and quality indexes in line with the quality control standard for the Liang Di Tang preparation, with small differences (for the dry extract yields, paeoniflorin contents, and harpagoside contents of the three batches of finished products, RSD was less than 5%). Results are shown in Table 14.

**Table 14 Experimental results of the batches of commercial-scale production testing**

| Batch No. | Finishe d product amount (kg) | Dry extract yield (%) | Harpago side content (mg/bag ) | Paeoniflor in content (mg/bag) | L-hydroxyproli ne content (mg/bag) | Glycine content (mg/bag) | Characterist ic chromatogra m |
|---|---|---|---|---|---|---|---|
| 211200 2 | 395.9 | 46.94 | 6.9 | 44.3 | 138.2 | 272.1 | Meeting the requirement s |
| 211200 3 | 426.0 | 49.92 | 6.8 | 44.1 | 136.2 | 267.7 | Meeting the requirement s |
| 211200 4 | 424.2 | 49.72 | 6.5 | 41.4 | 137.4 | 269.9 | Meeting the requirement s |
| Liang Di Tang referen ce sample | - | 42-52 | 4.0-8.0 | 33.0-62.0 | 100.0-200.0 | 210.0-390 .0 | 0.22 (peak 1), 0.26 (peak 2), 0.31 (peak 3), 0.33 (peak 4), 1.00 (peak 5), 1.47 (peak 6), 1.54 (peak 7), 1.82 (peak 8), 1.91 (peak 9), and 2.22 (peak 10) |

### Example 7

### 1. Three batches of pilot testings (mixed concentration tests) (batch numbers: 2105012, 2105013, and 2106014)

### 1.1 Experimental methods for the three batches of pilot testings

12.309 kg of wine-processed *Rehmanniae Radix,* 12.309 kg of *Scrophulariae Radix,* 6.138 kg of wine-processed *Paeoniae Radix Alba,* 6.138 kg of *Ophiopogon japonicus,* and 3.696 kg of *Lycii Cortex* were weighed. Water was added at an amount 7 times an amount of the Chinese herbal slices, and soaking was conducted for 40 min. The resulting mixture was subjected to a first heat extraction at 100 °C for 30 min, and a resulting medicinal liquid was discharged. Water was then added at an amount 5 times the amount of the Chinese herbal slices to the residue, a resulting mixture was subjected to a second heat extraction at 100 °C for 20 min, and a resulting medicinal liquid was discharged. Water was added at an amount 5 times the amount of the Chinese herbal slices to the residue, a resulting mixture was subjected to a third heat extraction at 100 °C for 20 min, and a resulting medicinal liquid was discharged. The three medicinal liquids produced each were measured and tested for a density, a dry extract yield, and harpagoside and paeoniflorin contents.

Each medicinal liquid was filtered through a plate and frame filter (a filter membrane had a pore size of 5 µm to 15 µm and tested for a density (when a pressure exceeded 0.02 MPa, a fine filter plate was replaced). The number of plate replacements was recorded.

3.696 kg of *Asini Corii Colla* was weighed and processed into a powder. Water was added at an amount about 6 times an amount of the *Asini Corii Colla,* and a resulting mixture was heated until boiling, with a continuous stirring, to obtain a homogeneous *Asini Corii Colla* liquid. The homogeneous *Asini Corii Colla* liquid was further concentrated to densities of 1.16 g/mL, 1.18 g/mL, and 1.17 g/mL.

2.4 kg of dextrin was weighed and poured into a stainless-steel barrel, water was added in a mass 4 times a mass of the dextrin, and a resulting mixture was stirred until complete dissolution to obtain a dextrin solution.

Under vacuum, each medicinal liquid produced after filtration was fed into a vacuum concentrator. A steam valve was opened, and heating was started. Vacuum concentration was conducted at a temperature of 85±5 °C, a vacuum degree of 0.045±0.015 MPa, and a steam pressure of 0.025±0.005 MPa to obtain a concentrated medicinal liquid with a density of about 1.20 g/mL. During the concentration, a boiling state and a temperature were observed at any time, and the vacuum degree and the steam pressure were adjusted. The *Asini Corii Colla* liquid and the dextrin solution were fed into the vacuum concentrator. A resulting mixture was further vacuum concentrated to obtain a thick paste with a density of 1.30 g/mL to 1.35 g/mL (20 °C). The thick paste was transferred into a transfer barrel for later use. The thick paste was weighed and tested for a density, a dry extract yield, and harpagoside and paeoniflorin contents.

An induction cooker was turned on to slowly heat a medicinal liquid (which was intended to prevent the gelatinization of the medicinal liquid and the destruction of ingredients), under a continuous stirring to maintain excellent fluidity of the medicinal liquid. A heating power was adjusted to keep the medicinal liquid warm. Drying was conducted in a vacuum belt dryer to obtain a powder. Parameters of the vacuum belt dryer were as follows: a feeding rate: 1.5 L/h; a running speed of a track: 160 mm/min; an angle of a swingarm: 26°; a speed of a material-distributing motor: 100 r/min; heating temperatures in a first zone, a second zone, a third zone, and a fourth zone being: 95±5 °C, 95±5 °C, 95±5 °C, and 30±5 °C, respectively; a speed of a cutter: 10 s/time; and a vacuum degree: 0.0985±0.0015 MPa. The powder was collected in a transfer barrel and packaged for later use. The powder produced after the belt drying was weighed and tested for harpagoside and paeoniflorin contents.

About 1.5 kg of the powder produced after the belt drying was taken, weighed, added to a hopper of a granulator, and granulated. Parameters for the granulator were as follows: a pressure of a press roller: 10.0 MPa; rotational speeds of a screw feeder: a vertical rotational speed being 45 r/min, and a horizontal rotational speed being 120 r/min; a rotational speed of the press roller: 8.0 r/min; and a mesh size of a sieve: 16 mesh. During the granulation, the conditions of the granulator and the appearances of particles were observed, the granulation parameters were adjusted appropriately, and the powder was fed and the particles were collected in time. Screening was conducted. A primary formation rate and a fine rate were calculated.

### 1.2 Experimental results

Based on the experimental conditions and detection results of the three batches of pilot testings for the Liang Di Tang granule, the mass transfer analysis was conducted for each batch. Mass transfer data for the three batches of pilot testings are shown in Table 15 and FIG. 10.

**Table 15 Average mass transfer analysis results for the three batches of pilot testings for the Liang Di Tang granule**

| No. | Process stage | Dry extract yield/% | Harpagoside transfer rate/% | Paeoniflorin transfer rate/% |
|---|---|---|---|---|
| 1 | Extraction | 54.72 | 73.08 | 68.91 |
| 2 | Vacuum concentration | 51.10 | 65.38 | 52.72 |
| 3 | Dry powder | 44.24 | 53.2 | 43.27 |
| | Accompanying decoction | 36.8 | 48.08 | 60.15 |

### Analysis results:

(1) During the extraction process, an average extract yield is 54.72%, a paeoniflorin transfer rate is 73.08, and a harpagoside transfer rate is 68.91, which all reach or exceed the respective levels of the accompanying decoction and the reference sample. It indicates that the extraction meets the requirements of the development of the classical formula.
(2) During a process from extraction to concentration and drying, the dry extract yield and the harpagoside and paeoniflorin are reduced to varying degrees. Losses of the components are greater than a loss of the dry extract yield. According to analysis results, the losses during the process are mainly concentrated in the vacuum concentration and drying: 1) vacuum concentration: losses of paeoniflorin and harpagoside are 16.35% and 6.90% more than a loss of a dry extract yield, respectively, indicating that the index components, especially paeoniflorin, are partially destructed during the concentration., indicating that the concentration is a major procedure for the losses of the index components; 2) drying: losses of paeoniflorin and harpagoside are consistent with a loss of the dry extract yield, which are 12.52%, 11.49%, and 11.98%, respectively, with losses being mainly attributed to the residue in containers, pipelines, and devices.
(3) Based on the analysis of the major parameters such as a temperature and a time for vacuum concentration and the experimental research on the stability of paeoniflorin, it is believed that paeoniflorin in a Liang Di Tang medicinal liquid is easily decomposed during the vacuum concentration (temperature: 80 °C to 90 °C) at a pH value of higher than 6. According to detection results, a higher concentration of an extraction solution results in a lower pH value. The first and second extraction solutions have a relatively low pH value, and the third extraction solution has a pH value of higher than 6. Therefore, it is recommended that the three extraction solutions produced in the production process of the Liang Di Tang granule should be concentrated separately, then mixed, and concentrated, which could minimize the time of heating paeoniflorin at a low concentration.

### 2. Three batches of pilot testings after optimization (separate concentration + mixed concentration tests) (batch numbers: 2107015, 2107016, and 2107017)

### 2.1 Experimental method

12.309 kg of wine-processed *Rehmanniae Radix,* 12.309 kg of *Scrophulariae Radix,* 6.138 kg of wine-processed *Paeoniae Radix Alba,* 6.138 kg of *Ophiopogon japonicus,* and 3.696 kg of *Lycii Cortex* were weighed. Water was added at an amount 7 times an amount of the Chinese herbal slices, and soaking was conducted for 40 min. A resulting mixture was subjected to a first heat extraction at 100 °C for 30 min, and a resulting medicinal liquid was discharged and subjected to fine filtration. Water was added at an amount 5 times the amount of the Chinese herbal slices to the residue. A resulting mixture was subjected to a second heat extraction at 100 °C for 20 min, and a resulting medicinal liquid was discharged and subjected to fine filtration. Water was added at an amount 5 times the amount of the Chinese herbal slices to the residue. A resulting mixture was subjected to a third heat extraction at 100 °C for 20 min, and a resulting medicinal liquid was discharged and subjected to fine filtration. The three medicinal liquids produced were tested for a density, a dry extract yield, and harpagoside and paeoniflorin contents.

Each medicinal liquid was filtered through a plate and frame filter (a filter membrane had a pore size of 5 µm to 15 µm and tested for a density (when a pressure exceeded 0.02 MPa, a fine filter plate was replaced). The number of plate replacements was recorded.

Under vacuum, a first filtrate was fed in a vacuum concentrator, a steam valve was opened, and heating was started. The first filtrate was concentrated at a temperature of 85±5 °C, a vacuum degree of 0.04 ± 0.01 MPa, and a steam pressure of 0.025±0.005 MPa to obtain about 20 L of a concentrate (density: about 1.18 g/mL), and the concentrate was transferred into a storage tank for later use. A second filtrate was fed in a vacuum concentrator, a steam valve was opened, and heating was started. The second filtrate was concentrated under the same conditions as described above for the first filtrate to obtain about 20 L of a concentrate (density: about 1.10 g/mL), and the concentrate was transferred into a storage tank for later use. A third filtrate was fed in a vacuum concentrator, a steam valve was opened, and heating was started. The third filtrate was concentrated under the same conditions as described above for the first filtrate to obtain about 20 L of a concentrate (density: about 1.04 g/mL), and the concentrate was transferred into a storage tank for later use.

3.696 kg of *Asini Corii Colla* was weighed and processed into a powder. Water was added in a mass 3 times a mass of the *Asini Corii Colla,* a resulting mixture was heated until boiling, with a continuous stirring, to obtain a homogeneous *Asini Corii Colla* liquid. The homogeneous *Asini Corii Colla* liquid was further concentrated to a density of larger than or equal to 1.16 g/mL to obtain an *Asini Corii Colla* liquid.

About 2.4 kg of dextrin was weighed and poured into a stainless-steel barrel, water was added in a mass 1 time to 2 times a mass of the dextrin, and a resulting mixture was stirred until complete dissolution to obtain a dextrin solution.

Under vacuum, the three concentrates, the *Asini Corii Colla* liquid, and the dextrin solution were fed in a vacuum concentration tank, a steam valve was opened, and vacuum concentration was conducted at a temperature of 85±5 °C, a vacuum degree of 0.04±0.01 MPa, and a steam pressure of 0.025±0.005 MPa to obtain a thick paste with a density of about 1.30 g/mL (20 °C). The thick paste was filtered through a filter and then transferred into a transfer barrel for later use. Requirement: A time interval between medicinal liquid discharge and drying shall not exceed 8 h.

An induction cooker was turned on to slowly heat a medicinal liquid (which was intended to prevent the gelatinization of the medicinal liquid and the destruction of ingredients), under a continuous stirring to maintain excellent fluidity of the medicinal liquid. A heating power was adjusted to keep the medicinal liquid warm. Drying was conducted in a vacuum belt dryer to obtain a powder. Parameters of the vacuum belt dryer were as follows: a feeding rate: 1.5 L/h; a running speed of a track: 160 mm/min; an angle of a swingarm: 26°; a speed of a material-distributing motor: 100 r/min; heating temperatures in a first zone, a second zone, a third zone, and a fourth zone being: 95±5 °C, 95±5 °C, 95±5 °C, and 30±5 °C, respectively; a speed of a cutter: 10 s/time; and a vacuum degree: 0.0985±0.0015 MPa. The powder was collected in a transfer barrel and packaged for later use.

About 1.5 kg of the powder produced after the belt drying was taken, weighed, added to a hopper of a granulator, and granulated. Parameters for the granulator were as follows: a pressure of a press roller: 10.0 MPa; rotational speeds of a screw feeder: a vertical rotational speed being 45 r/min, and a horizontal rotational speed being 120 r/min; a rotational speed of the press roller: 8.0 r/min; and a mesh size of a sieve: 16 mesh. During the granulation, the conditions of the granulator and the appearances of particles were observed (when particles were loose and excessively fine, the pressure of the press roller was increased and the rotational speed of the feeder was reduced; when particles were hard, the pressure of the press roller was decreased and the rotational speed of the feeder was reduced), and the powder was fed and the particles were collected in time.

### 2.2 Experimental results

According to the experimental conditions and detection results of the three batches of pilot testings for the Liang Di Tang granule, a dry extract yield, a harpagoside transfer rate, and a paeoniflorin transfer rate were taken as investigation indexes to analyze the mass transfer in a test process. Changes of the investigation indexes in the extraction, separate concentration, mixed concentration, and drying procedures for the Liang Di Tang granule were calculated and analyzed. In the processes of the three batches of pilot testings, the average mass transfer conditions are shown in Table 16 (data was an average of the three batches of pilot testings), and the losses (a loss rate was calculated by dividing a loss by a total extract amount, and data was an average of the three batches of pilot testings) are shown in Table 17.

**Table 16 Analysis results of mass transfer in the three batches of pilot testings for the Liang Di Tang granule**

| No. | Process stage | Dry extract yield/% | Harpagoside transfer rate/% | Paeoniflorin transfer rate/% |
|---|---|---|---|---|
| 1 | Extraction | 50.73 | 75.28 | 61.37 |
| 2 | Separate concentration | 52.28 | 78.40 | 62.47 |
| 3 | Mixed concentration | 52.10 | 73.73 | 59.44 |
| 4 | Dry powder | 41.20 | 62.13 | 48.42 |
| Accompanying decoction | ---- | 42.83 | 49.33 | 48.97 |

**Table 17 Analysis results of losses of quality index components in the three batches of pilot testings for the Liang Di Tang granule**

| Process stage | Extract loss rate/% | Harpagoside loss rate/% | Paeoniflorin loss rate/% |
|---|---|---|---|
| Concentration | 4.45 | 2.40 | 5.42 |
| Belt drying | 16.66 | 15.27 | 18.25 |
| Total | 21.11 | 17.67 | 23.67 |

| | | | |
|---|---|---|---|
| Note: Losses are mainly concentrated in the belt drying procedure. In the different batches of belt drying, a loss in the dry extract yield is consistent with losses of the components. | | | |

Description: (1) dry extract yields and paeoniflorin transfer rates of the three batches of pilot testings (2107015, 2107016, and 2107017) are basically consistent, where the dry extract yields are 49.66%, 51.43%, and 49.66%, respectively and the paeoniflorin transfer rates are 60.63%, 63.47%, and 60.00%, respectively. Harpagoside transfer rates of the batches 2107015 and 2107017 are basically consistent, being 84.69% and 84.00%, respectively. The harpagoside transfer rate of the batch 2107016 is relatively low, being 57.14%, which is caused by the non-uniformity of the Chinese herbal slices of *Scrophulariae Radix.*

(2) Analysis of losses of the major quality indexes from an extraction solution to a dry powder in the three batches of pilot testings: 1) The dry extract yield and the harpagoside and paeoniflorin all undergo a partial loss (average losses are 21.12%, 17.68%, and 23.67%, respectively), and loss rates are basically consistent. According to analysis results, it is believed that the losses are mainly caused by the losses of materials, such as the residue in pipelines and devices. 2) At a concentration stage, major quality indexes such as the dry extract yield and the paeoniflorin are lost by 4.45% and 5.42%, respectively, and a destruction rate of paeoniflorin is 1.0%, therby demonstrating that the separate concentration solves the problem of paeoniflorin destruction during concentration.

(3) From the Chinese herbal slices to the dry powder, the average dry extract yield, harpagoside transfer rate, and paeoniflorin transfer rate for the three batches of pilot testings are basically consistent with those of the accompanying decoction, which are 41.20%, 62.13%, and 48.4%, in comparsion with 42.83%, 49.33%, and 48.97%, respectively. It indicates that the pilot process for the Liang Di Tang granule meets the requirements of the classical formula (the preparation has quality indexes consistent with the reference material). It has been validated that the optimized small-scale process for the Liang Di Tang Granule is suitable for the modern traditional Chinese medicine preparation production.

### 3. Conclusion of a contrast test between mixed concentration and separate concentration + mixed concentration

(1) Mixed concentration: A mixed medicinal liquid of the three medicinal liquids had a pH of higher than 6.0. A heating time was as long as 2 h to 4 h. A resulting concentrate had a low concentration (lower than 0.5 mg/mL). A destruction rate of paeoniflorin was 18.6%.
(2) Separate concentration + mixed concentration: The first and second extraction solutions had a pH value of lower than 6.0, and the third extraction solution had a pH value of higher than 6.0. A heating time was shortened to less than 2 h. A concentration of a resulting concentrate was increased (higher than 0.5 mg/mL). A destruction rate of paeoniflorin was 1.2%.

Based on the principle of "seeking common ground while reserving differences, and honoring tradition without being confined by it", the present disclosure conducts the textual research on the formula, sources, processing, decoction, and medication practices, and establishes a preparation process and quality standard for the Liang Di Tang reference sample through experimental researches, so as to achieve the solidification and controllability of a decoction of "Liang Di Tang". The modern industrial preparation devices are adopted for extraction, refinement, concentration, drying, and granulation, and the modern analytical techniques such as HPLC are adopted to establish methods for determining contents of various components and for measuring a characteristic chromatogram. Chemical component changes before and after each step during commercial production are monitored, and the mass transfer is analyzed, which ensures the consistency in chemical components between industrial preparations and decoctions prepared by the traditional technology. In addition, the Liang Di Tang granule has advantages such as high stability, portability, and convenient taking over the traditional decoction.

In the present disclosure, a modern preparation of Liang Di Tang is determined through experiments. Chinese herbal slices and an adjuvant for preparing 1,000 g of the Liang Di Tang granule are as follows: 520 g of wine-processed *Rehmanniae Radix,* 520 g of *Scrophulariae Radix,* 260 g of wine-processed *Paeoniae Radix Alba,* 260 g of *Ophiopogon japonicus,* 156 g of *Lycii Cortex,* 156 g of *Asini Corii Colla,* and 100 g to 150 g of dextrin.

In the present disclosure, when the extraction process is optimized, a Liang Di Tang reference sample is taken as a benchmark (an extract yield, paeoniflorin and harpagoside contents, and a characteristic chromatogram are taken as measure indexes). A preliminary test is first conducted under the conditions of pilot testing production, and it is found that an extraction rate of the modern preparation (involving an extract yield and paeoniflorin and harpagoside contents) is 20% to 30% higher than that of the accompanying decoction (a decoction prepared by a preparation method for a reference sample from the same batch of Chinese herbal slices in the preliminary test). The standards of the reference sample then could be met even with the losses in procedures such as refinement, concentration, and drying accounted. Based on the improvement of an extraction rate by 20% to 30%, the extraction processes for the Liang Di Tang granule are screened. The determined extraction process has a moderate water amount, a short time, and a high extraction efficiency (the extract yield and the paeoniflorin and harpagoside contents all are high). In the existing modern extraction modes for Chinese patent drugs where the extraction rate is blindly pursued (The extraction each time is often conducted for 1 h to 2 h. However, the extracted components are often some impurities such as fibers and starches, and the extraction of effective ingredients is not improved. Moreover, the long-term heating may even cause the destruction of some ingredients) and involves excessive extraction and have high energy consumption, while the extraction process of the present disclosure improves the quality and efficacy of a product and reduces the production cost.

The whole process after the extraction in the present disclosure is carried out at a low temperature (lower than 100 °C). During the refinement, the physical modes of centrifugation and plate and frame filtration are adopted to remove impurities. A vacuum concentration mode is adopted for concentration. A vacuum belt drying mode is adopted for drying. The low temperature is conducive to the protection and retention of components, and could avoid the destruction of components due to high temperature. Thus, the destruction during the industrial preparation production process is minimized, and the consistency between the traditional decoction and the modern preparation is guaranteed.

The vacuum concentration process adopted in the present disclosure is different from the traditional vacuum concentration process for Chinese patent drugs. It has been found through researches that paeoniflorin in a low-concentration Liang Di Tang medicinal liquid is easily destroyed when heated for a long time. In the traditional concentration for Chinese patent drugs, the first extraction solution is fed into a vacuum concentration tank and vacuum-concentrated, and the second and third extraction solutions are sequentially fed into the vacuum concentration tank and further concentrated to a specified density. This concentration mode reduces the total concentration of a medicinal liquid and prolongs the absolute heating and concentration time for the medicinal liquid. The present disclosure adopts a gradient concentration mode of separate concentration + mixed concentration, where the first, second, and third extraction solutions are concentrated separately to a specified density, and then mixed and concentrated to a specified density. This concentration mode makes a content of paeoniflorin in each of the three extraction solutions quickly reach a high level, and allows a short low-content concentration time (an environment where paeoniflorin is easily destructed). Thus, the present disclosure finds the optimal concentration process conditions for the Liang Di Tang granule and solves the problem of destruction of the pharmacodynamic component paeoniflorin in the Liang Di Tang granule. According to the researches on the stability of paeoniflorin, when vacuum concentration is conducted at a temperature of 80 °C to 90 °C for a Liang Di Tang medicinal liquid with a paeoniflorin concentration of lower than 0.3 mg/mL and a pH value of higher than 6.0, paeoniflorin is partially destroyed. The third extraction solution for the Liang Di Tang granule has a low paeoniflorin concentration (lower than 0.04 mg/mL). If the three extraction solutions are directly mixed and concentrated according to the traditional concentration mode for traditional Chinese medicine, a resulting mixed medicinal liquid would have a low paeoniflorin concentration and a high pH value (higher than 6.0), and paeoniflorin undergoes partial destruction under conditions of a low concentration, a high pH value, and a long thermal concentration time. According to tests, the three extraction solutions for the Liang Di Tang granule are centrifuged and subjected to fine filtration separately, and resulting filtrates are fed into a vacuum concentrator and then vacuum-concentrated (a temperature: 80 °C to 90 °C, a vacuum degree: 0.03 MPa to 0.06 MPa, and a steam pressure: 0.03 MPa to 0.05 MPa) until a density is larger than or equal to 1.10 g/mL to obtain a medicinal concentrate with a paeoniflorin content of higher than or equal to 0.3 mg/mL and a pH value of lower than 6.0, which reaches a stable state.

In the preparation process of the Liang Di Tang preparation of the present disclosure, the adjuvant of dextrin is added during concentration. That is to say, the adjuvant of dextrin is dissolved in water, then introduced into a vacuum concentrator, mixed with a concentrated medicinal liquid and an *Asini Corii Colla* liquid, and concentrated, which solves the problem that there is a material loss during a vacuum belt drying process because a medicinal liquid of the Liang Di Tang granule is viscous (containing *Asini Corii Colla* and polysaccharides) and prone to form bubble. Moreover, compared with the conventional addition of an adjuvant before granulation, the addition mode of the present disclosure could solve the problem of thorough mixing between an adjuvant and raw materials in the modern granule.

Because Liang Di Tang includes heat-sensitive ingredients such as *Asini Corii Colla* (highly viscous and easy to agglomerate), polysaccharides, and paeoniflorin, the traditional high-temperature drying and spray-drying modes are easy to cause agglomeration and may cause destruction to heat-sensitive active ingredients such as paeoniflorin. The present disclosure adopts a vacuum belt drying mode, which has advantages such as low drying temperature, short drying time, small loss, and high efficiency. During the drying process, the Liang Di Tang preparation is not prone to agglomeration, and the heat-sensitive ingredients such as paeoniflorin are not destroyed. As a result, the components are evenly distributed in the Liang Di Tang preparation, and the Liang Di Tang preparation has high contents of active ingredients such as paeoniflorin and exhibits prominent efficacy.

The quality standard for the Liang Di Tang preparation provided by the present disclosure stipulate the upper and lower limits for harpagoside and paeoniflorin contents. In the present disclosure, after the researches on the preparation process for the reference sample is completed, the quality standard for the Liang Di Tang preparation is formed by establishing an overall quality control mode with a reference sample as a benchmark, and comprehensively monitoring the overall chemical composition of the reference sample using index component contents + characteristic chromatogram technique. The established multi-component control index is used for investigating the stability of products to ensure the commercialization of finished drugs. Liang Di Tang is an ancient classical formula that could be prepared with the modern pharmaceutical equipment and technology. In the present disclosure, the extraction, concentration, drying, and granulation processes are optimized to prepare a modern preparation, and a "medicines-Chinese herbal slices-preparation" whole-process quality control system is established to ensure that the modern preparation maintains the consistency with the traditional decoction in terms of the material basis and the stability, and also has advantages of modern preparations, such as stable quality, portability, and convenient taking.

The above are merely preferred embodiments of the present disclosure. It should be noted that a person of ordinary skill in the art may further make several improvements and modifications without departing from the principle of the present disclosure, but such improvements and modifications should be deemed as falling within the scope of the present disclosure.

## Claims

1. A method for preparing a Liang Di Tang water-extracted liquid, comprising the steps of:
mixing five traditional Chinese medicines with a first water, and conducting a first soaking and then a first extraction to obtain a first extraction solution and a first medicinal residue, wherein the five traditional Chinese medicines are wine-processed *Rehmanniae Radix, Scrophulariae Radix,* wine-processed *Paeoniae Radix Alba, Ophiopogon japonicus,* and *Lycii Cortex;*
mixing the first medicinal residue with a second water, and conducting a second soaking and then a second extraction to obtain a second extraction solution and a second medicinal residue;
mixing the second medicinal residue with a third water, and conducting a third soaking and then a third extraction to obtain a third extraction solution;
concentrating the first extraction solution, the second extraction solution, and the third extraction solution separately to obtain a first concentrate, a second concentrate, and a third concentrate, respectively, wherein the concentrating is conducted at a temperature independently of 70 °C to 90 °C, a vacuum degree independently of 0.03 MPa to 0.07 MPa, and a steam pressure independently of not larger than 0.09 MPa; and
mixing the first concentrate, the second concentrate, the third concentrate, and an *Asini Corii Colla* liquid to obtain the Liang Di Tang water-extracted liquid,
wherein a mass ratio of the wine-processed *Rehmanniae Radix,* the *Scrophulariae Radix,* the wine-processed *Paeoniae Radix Alba,* the *Ophiopogon japonicus,* the *Lycii Cortex,* and *Asini Corii Colla* is 37.30 : 37.30 :18.65 : 18.65 : 11.19 : 11.19.

2. The method for preparing the Liang Di Tang water-extracted liquid as claimed in claim 1, wherein a mass of the first water is 6 times to 8 times a total mass of the five traditional Chinese medicines;
a mass of the second water and a mass of the third water each are independently 4 times to 6 times the total mass of the five traditional Chinese medicines;
the first extraction, the second extraction, and the third extraction each are conducted independently at a temperature of 98 °C to 101 °C;
the first soaking and the first extraction each are conducted independently for 30 minutes to 50 minutes; and
the second soaking, the second extraction, the third soaking, and the third extraction each are conducted independently for 20 minutes to 40 minutes.

3. The method for preparing the Liang Di Tang water-extracted liquid as claimed in claim 1 or 2, further comprising:
subjecting a first extraction system obtained after the first extraction to post-treatment, wherein the post-treatment comprises: subjecting the first extraction system to centrifugal separation to obtain liquid components and the first medicinal residue, and subjecting the liquid components to fine filtration to obtain the first extraction solution.

4. The method for preparing the Liang Di Tang water-extracted liquid as claimed in claim 3, wherein the fine filtration is conducted with a plate and frame filter, and a filter membrane of the plate and frame filter has a pore size of 5 µm to 15 µm.

5. The method for preparing the Liang Di Tang water-extracted liquid as claimed in claim 1, wherein the first concentrate, the second concentrate, and the third concentrate each have a density independently of greater than or equal to 1.10 g/mL and less than 1.26 g/mL.

6. The method for preparing the Liang Di Tang water-extracted liquid as claimed in claim 1, wherein the *Asini Corii Colla* liquid is prepared by a process comprising: placing the *Asini Corii Colla* in water, liquefying the *Asini Corii Colla,* and sieving to obtain an undersized part, namely the *Asini Corii Colla* liquid, wherein a mass of the water is 2 times to 6 times a mass of the *Asini Corii Colla,* and a sieve used for the sieving has a sieve size of 100 mesh.

7. A Liang Di Tang water-extracted liquid prepared by the method for preparing the Liang Di Tang water-extracted liquid as claimed in any one of claims 1 to 6.

8. A Liang Di Tang water-extracted paste obtained by concentrating the Liang Di Tang water-extracted liquid as claimed in claim 7, wherein the Liang Di Tang water-extracted paste has a density of 1.2 g/mL to 1.3 g/mL.

9. A Liang Di Tang preparation prepared from raw materials comprising a Liang Di Tang water-extracted extract and a pharmaceutically acceptable adjuvant, wherein
the Liang Di Tang water-extracted extract is at least one selected from the group consisting of the Liang Di Tang water-extracted liquid as claimed in claim 7 and the Liang Di Tang water-extracted paste as claimed in claim 8;
a mass ratio of the pharmaceutically acceptable adjuvant to the Liang Di Tang water-extracted extract is in a range of 1 : 12.48 to 1 : 18.72, and
a mass of the Liang Di Tang water-extracted extract is based on a total mass of wine-processed *Rehmanniae Radix, Scrophulariae Radix,* wine-processed *Paeoniae Radix Alba, Ophiopogon japonicus, Lycii Cortex,* and *Asini Corii Colla.*

10. The Liang Di Tang preparation as claimed in claim 9, wherein in parts by mass, the raw materials for preparing 1,000 parts of the Liang Di Tang preparation comprise: 468 parts to 572 parts of the wine-processed *Rehmanniae Radix,* 468 parts to 572 parts of the *Scrophulariae Radix,* 234 parts to 286 parts of the wine-processed *Paeoniae Radix Alba,* 234 parts to 286 parts of the *Ophiopogon japonicus,* 140 parts to 171 parts of the *Lycii Cortex,* 140 parts to 171 parts of the *Asini Corii Colla,* and 100 parts to 150 parts of the pharmaceutically acceptable adjuvant.

11. The Liang Di Tang preparation as claimed in claim 9 or 10, wherein a water content in the Liang Di Tang preparation is in a range of 3 wt% to 8 wt%.

12. The Liang Di Tang preparation as claimed in claim 9 or 10, wherein the Liang Di Tang preparation comprises a Liang Di Tang powder or a Liang Di Tang granule; and the Liang Di Tang powder has a particle size of 150 µm to 850 µm and the Liang Di Tang granule has a particle size of 180 µm to 2,000 µm.

13. A method for preparing the Liang Di Tang preparation as claimed in any one of claims 9 to 12, comprising the steps of:
mixing and concentrating the Liang Di Tang water-extracted extract and an aqueous solution of the pharmaceutically acceptable adjuvant to obtain a mixed concentrate; and
subjecting the mixed concentrate to vacuum belt drying to obtain the Liang Di Tang preparation.

14. The method for preparing the Liang Di Tang preparation as claimed in claim 13, wherein the aqueous solution of the pharmaceutically acceptable adjuvant is obtained by dissolving the pharmaceutically acceptable adjuvant in water, and a mass ratio of the pharmaceutically acceptable adjuvant to the water is in a range of 1 : 2 to 1 : 6.

15. The method for preparing the Liang Di Tang preparation as claimed in claim 13, wherein the concentrating is conducted at a temperature of 70 °C to 90 °C, a vacuum degree of 0.03 MPa to 0.07 MPa, and a steam pressure of not larger than 0.09 MPa.

16. The method for preparing the Liang Di Tang preparation as claimed in claim 13 or 15, wherein the mixed concentrate has a density of 1.25 g/mL to 1.36 g/mL.

17. The method for preparing the Liang Di Tang preparation as claimed in claim 13, wherein the vacuum belt drying is conducted in a vacuum belt dryer, and parameters for the vacuum belt drying comprise: a feeding temperature of 75 °C to 85 °C; a feeding rate of 6 L/h to 24 L/h; a running speed of a track being 16±5 cm/min; an angle of a swingarm of 65° to 85°; a speed of a material-distributing motor being 30 r/min to 50 r/min; a heating temperature in a first zone of 95±5 °C, a heating temperature in a second zone of 96±5 °C, a heating temperature a third zone of 96±5 °C, and a heating temperature in a fourth zone of 30±5 °C; a speed of a cutter being 10 s/time to 20 s/time; and a vacuum degree of 0.097 MP to 0.1 MP.

18. The method for preparing the Liang Di Tang preparation as claimed in claim 13 or 17, wherein under the condition that the Liang Di Tang preparation is the Liang Di Tang granule, the method further comprises:
conducting granulation after the vacuum belt drying to obtain the Liang Di Tang granule; and
the granulation is conducted using a granulator, and parameters for the granulation comprise: a pressure of a press roller being 12 MPa to 20 MPa; a vertical rotational speed of a screw feeder being 18 r/min to 30 r/min; a horizontal rotational speed of the screw feeder being 90 r/min to 140 r/min; a rotational speed of the press roller being 4 r/min to 10 r/min; and a granule sizing speed of 100 r/min to 150 r/min.

19. A quality control standard for the Liang Di Tang preparation as claimed in any one of claims 9 to 12 or the Liang Di Tang preparation prepared by the method for preparing the Liang Di Tang preparation as claimed in any one of claims 13 to 18, comprising a high-performance liquid chromatography (HPLC) characteristic chromatogram, characteristic component contents, a water content, and a dry extract yield of the Liang Di Tang preparation;
the characteristic component contents comprise: a harpagoside content of 0.33 mg/g to 0.67 mg/g, a paeoniflorin content of 2.62 mg/g to 4.96 mg/g, an L-hydroxyproline content of 8.83 mg/g to 16.42 mg/g, and a glycine content of 17.42 mg/g to 32.46 mg/g;
the water content ranges from 3 wt% to 8 wt%; and
the dry extract yield ranges from 40% to 55%.

20. The quality control standard as claimed in claim 19, wherein the HPLC characteristic chromatogram of the Liang Di Tang preparation is acquired by a process comprising the steps of:
(1) mixing and concentrating the Liang Di Tang preparation to be tested, diatomaceous earth, and an aqueous acetonitrile solution to obtain a concentrate, and dissolving the concentrate in an aqueous methanol solution to obtain a test sample solution;
(2) mixing a paeoniflorin reference substance, a harpagoside reference substance, and methanol to obtain a reference substance solution; and
(3) subjecting the test sample solution and the reference substance solution to HPLC test separately to obtain a reference substance characteristic chromatogram and a test sample characteristic chromatogram; and based on a retention time of the paeoniflorin reference substance in the reference substance characteristic chromatogram, calculating a relative retention time of each of other characteristic peaks in the test sample characteristic chromatogram to obtain the HPLC characteristic chromatogram of the Liang Di Tang preparation,
wherein step (1) and step (2) are conducted in any order.

21. The quality control standard as claimed in claim 20, wherein conditions for the HPLC test comprise: adopting a chromatographic column with octadecylsilane-bonded silica gel as a filler; a mobile phase A being methanol, a mobile phase B being a 0.05 v/v% phosphoric acid solution, and a mobile phase flow rate ranging from 0.8 mL/min to 1.2 mL/min; an elution mode of gradient elution, and a program for the gradient elution being as follows:
| time (minute) | volume fraction of the mobile phase A (%) | volume fraction of the mobile phase B (%) |
|---|---|---|
| 0-10 | 5→23.5 | 95→76.5 |
| 10-20 | 23.5 | 76.5 |
| 20-58 | 23.5→63 | 76.5→37 |
| 58-60 | 63→90 | 37→10 |
a detection wavelength of 254 nm; a column temperature of 28 °C; and an injection sample volume of 10 µL.
